# EUROPEAN PATENT APPLICATION

(11) **EP 4 431 599 A1**
(43) Date of publication of application: **18.09.2024**
(21) Application number: 22890066.8
(22) Date of filing: 07.11.2022
(51) Int. Cl.: C12N 7/01, A61K 39/215, A61P 31/14, A61P 37/04, C07K 14/165, C12N 7/04, C12N 15/50

(54) **BETACORONAVIRUS ATTENUATED STRAIN**

(30) Priority: 08.11.2021 JP 2021182051; 24.08.2022 JP 2022133080
(71) Applicant: The Research Foundation for Microbial Diseases of Osaka University, Suita-shi, Osaka 565-0871 (JP)
(72) Inventor: TAKEKAWA, Shiro, Suita-shi, Osaka 565-0871 (JP); EBINA, Hirotaka, Suita-shi, Osaka 565-0871 (JP); OKAMURA, Shinya, San Francisco, California 94158 (US); YOSHIDA, Akiho, Suita-shi, Osaka 565-0871 (JP)
(74) Representative: Müller-Boré & Partner Patentanwälte PartG mbB
(86) International application number: PCT/JP2022/041445
(87) International publication number: WO 2023/080246

(57) **Abstract**

The purpose of the present invention is to provide a strain that is useful as a novel betacoronavirus vaccine. It is revealed that novel betacoronavirus, according to the present invention, having a prescribed substitution mutation relating to temperature sensitivity in combination with a prescribed deletion mutation relating to attenuation, is useful as a betacoronavirus vaccine strain having excellent attenuated characteristics.

## Description

### TECHNICAL FIELD

The present invention relates to a betacoronavirus attenuated strain.

### BACKGROUND ART

An infectious disease (COVID-19) with the novel coronavirus (SARS-CoV-2) has caused a pandemic and is still a social problem. As vaccines against this infectious disease, gene vaccines such as adenovirus vector vaccine and mRNA vaccines have been approved, and inoculation has been advanced all over the world, starting from Sputnik V (Non-Patent Document 1), which is an adenovirus vector vaccine approved in Russia.

### PRIOR ART DOCUMENT

### NON-PATENT DOCUMENT

Non-Patent Document 1: THE LANCET, VOLUME 396, ISSUE 10255, P 887-897, SEPTEMBER 26, 2020

### SUMMARY OF THE INVENTION

### PROBLEMS TO BE SOLVED BY THE INVENTION

However, gene vaccines are next-generation vaccines different from conventional vaccines, and side reactions such as fever and thrombosis have been reported. Therefore, it is considered that development of new vaccines is still important.

Therefore, it is an object of the present invention to provide a strain useful as a new betacoronavirus vaccine.

### MEANS FOR SOLVING THE PROBLEM

As a result of intensive studies, the present inventors have found that a novel betacoronavirus having a combination of a prescribed substitution mutation related to temperature sensitivity and a prescribed deletion mutation related to growth reduction or other attenuation as prescribed mutations related to attenuation is useful as a vaccine strain of the betacoronavirus having excellent attenuation. The present invention has been completed by further studies based on this knowledge. That is, the present invention provides the inventions of the following modes.
Item 1. A betacoronavirus attenuated strain containing:
   non-structural protein(s) having the following mutation of (b), a combination of the following mutations of (e) and (f), and/or the following mutation of (h); and
   structural protein(s), accessory protein(s), and/or non-structural protein(s) having the following mutations of (n), (o), and/or (r):
      (b) a mutation of an amino acid residue corresponding to leucine at the 445th position of the amino acid sequence set forth in SEQ ID NO: 1 in NSP3,
      (e) a mutation of an amino acid residue corresponding to glycine at the 248th position of the amino acid sequence set forth in SEQ ID NO: 2 in NSP14,
      (f) a mutation of an amino acid residue corresponding to glycine at the 416th position of the amino acid sequence set forth in SEQ ID NO: 2 in NSP14,
      (h) a mutation of an amino acid residue corresponding to valine at the 67th position of the amino acid sequence set forth in SEQ ID NO: 3 in NSP16,
      (n) a loss-of-function mutation in ORF8,
      (o) a deletion of an amino acid sequence corresponding to the 681st to 684th positions of the amino acid sequence set forth in SEQ ID NO: 4 in a spike, and
      (r) a deletion of an amino acid sequence corresponding to the 32nd to 39th positions of the amino acid sequence set forth in SEQ ID NO: 8 in NSP 1.
Item 2. The virus attenuated strain according to item 1, in which four types of mutations or combination of mutations are selected from six types of mutations or combination of mutations: the mutation of (b), the combination of the mutations of (e) and (f), the mutation of (h), the mutation of (n), the mutation of (o), and the mutation of (r).
Item 3. The virus attenuated strain according to item 1 or 2, in which one to two types of mutations or combination of mutations are selected from three types of mutations or combination of mutations: the mutation of (b), the combination of the mutations of (e) and (f), and the mutation of (h).
Item 4. The virus attenuated strain according to any of items 1 to 3, in which the structural protein further contains the following mutations of (p) and/or (q):
   (p) a mutation containing deletions of amino acid sequences corresponding to the 679th to 680th positions and the 685th to 686th positions of the amino acid sequence set forth in SEQ ID NO: 4 in a spike, and
   (q) a mutation of an amino acid residue corresponding to valine at the 687th position of the amino acid sequence set forth in SEQ ID NO: 4 in a spike.
Item 5. The virus attenuated strain according to item 4, containing the combination of the mutations of (e) and (f) and the following mutation of (g):
   (g) a mutation of an amino acid residue corresponding to alanine at the 504th position of the amino acid sequence set forth in SEQ ID NO: 2 in NSP14.
Item 6. The betacoronavirus attenuated strain according to item 4 or 5, in which the mutation of (n) is a deletion of an amino acid sequence corresponding to the amino acid sequence encoded by the base sequence set forth in SEQ ID NO: 7.
Item 7. The virus attenuated strain according to any of items 1 to 6, in which the mutation of (b) is a substitution with phenylalanine, the mutation of (e) is a substitution with valine, the mutation of (f) is a substitution with serine, and the mutation of (h) is a substitution with isoleucine.
Item 8. The virus attenuated strain according to any of items 4 to 7, in which the mutation of (q) is a substitution with isoleucine.
Item 9. The virus attenuated strain according to any of items 1 to 8, in which the betacoronavirus is SARS-CoV-2 virus.
Item 10. A live attenuated vaccine containing the virus attenuated strain according to any of items 1 to 9.
Item 11. The live attenuated vaccine according to item 10, which is administered nasally. Item 12. The live attenuated vaccine according to item 10, which is administered intramuscularly, subcutaneously, or intradermally.

### EFFECTS OF THE INVENTION

According to the present invention, a strain useful as a novel betacoronavirus vaccine is provided.

### BRIEF DESCRIPTION OF THE DRAWINGS

Fig. 1 shows a method for temperature sensitization of SARS-CoV-2.
Fig. 2 shows the results of confirmation (CPE images) of temperature sensitivity of SARS-CoV-2.
Fig. 3A shows the results of mutation analysis of each virus strain.
Fig. 3B shows CPE images by strains having a possibility of reverse mutation of a temperature-sensitive strain (A50-18 [Reference Example]).
Fig. 3C shows CPE images by strains having a possibility of reverse mutation of the temperature-sensitive strain (A50-18 [Reference Example]).
Fig. 3D shows the results of confirmation of temperature sensitivity of recombinant viruses into which mutations in the temperature-sensitive strain (A50-18 [Reference Example]) have been introduced.
Fig. 3E shows the results of confirmation of temperature sensitivity of recombinant viruses into which mutations in the temperature-sensitive strain (A50-18 [Reference Example]) have been introduced.
Fig. 4A shows the results of growth analysis of the temperature-sensitive strain (A50-18 [Reference Example]).
Fig. 4B shows the results of growth analysis of the temperature-sensitive strain (A50-18 [Reference Example]).
Fig. 5 shows weight changes of hamsters infected with the temperature-sensitive strain (A50-18 [Reference Example]).
Fig. 6 shows weight changes of hamsters infected with the temperature-sensitive strain (A50-18 [Reference Example]).
Fig. 7 shows viral amounts in the lungs or nasal wash of hamsters infected with the temperature-sensitive strain (A50-18 [Reference Example]).
Fig. 8 shows an image of the lungs of hamsters infected with the temperature-sensitive strain (A50-18 [Reference Example]).
Fig. 9 shows the results of histological analysis of the lungs of hamsters infected with the temperature-sensitive strain (A50-18 [Reference Example]).
Fig. 10 shows histological analysis (HE staining and IHC staining) of the lungs of hamsters infected with the temperature-sensitive strain (A50-18 [Reference Example]).
Fig. 11 shows weight changes of hamsters reinfected with the temperature-sensitive strain (A50-18 [Reference Example]).
Fig. 12 shows weight changes of hamsters after infection with the temperature-sensitive strain (A50-18 [Reference Example]).
Fig. 13 shows neutralizing antibody titers in serum of hamsters recovered after infection with the temperature-sensitive strain (A50-18 [Reference Example]).
Fig. 14 shows a method for temperature sensitization of SARS-CoV-2 (G to L50 series [Examples]).
Fig. 15 shows the results of confirmation (CPE images) of temperature sensitivity of SARS-CoV-2 (G to L50 series [Examples]).
Fig. 16A shows the results of mutation analysis of additional isolates (H50-11, L50-33, L50-40 [Examples]).
Fig. 16B shows CPE images of strains having a possibility of reverse mutation of the temperature-sensitive strain (H50-11 [Example]).
Fig. 16C shows CPE images of strains having a possibility of reverse mutation of the temperature-sensitive strains (L50-33, L50-40 [Examples]).
Fig. 17 shows deletions of base sequences found in relation to the temperature-sensitive strains (H50-11, L50-33, L50-40 [Examples]).
Fig. 18 shows a schematic overview of the deletions of the base sequences shown in Fig. 17 and deletions of amino acid sequences encoded thereby.
Fig. 19 shows the results of growth analysis of the temperature-sensitive strains (H50-11, L50-33, L50-40 [Examples]).
Fig. 20 shows weight changes of hamsters infected with the temperature-sensitive strains (A50-18 [Reference Example] and H50-11, L50-33, L50-40 [Examples]).
Fig. 21 shows lung weight of hamsters infected with the temperature-sensitive strains (A50-18 [Reference Example] and H50-11, L50-33, L50-40 [Examples]).
Fig. 22 shows viral amounts in the lungs or nasal wash of hamsters infected with the temperature-sensitive strains (A50-18 [Reference Example] and H50-11, L50-33, L50-40 [Examples]).
Fig. 23 shows weight changes of hamsters reinfected with the temperature-sensitive strains (A50-18 [Reference Example] and H50-11, L50-33, L50-40 [Examples]).
Fig. 24 shows neutralizing antibody titers in serum of hamsters after infection with the temperature-sensitive strains (A50-18 [Reference Example] and H50-11, L50-33, L50-40 [Examples]).
Fig. 25 shows evaluation of neutralizing activities of the temperature-sensitive strain (A50-18 strain [Reference Example]) against SARS-CoV-2 mutant strains.
Fig. 26 shows a comparison of immunogenicities between administration routes of the temperature-sensitive strain (A50-18 strain [Reference Example]).
Fig. 27 shows a comparison of immunogenicities between doses of the temperature-sensitive strain (A50-18 strain [Reference Example]).
Fig. 28 shows evaluation of neutralizing activities of the temperature-sensitive strain (A50-18 strain [Reference Example]) against SARS-CoV-2 mutant strains.
Fig. 29 shows evaluation of neutralizing activities of the temperature-sensitive strain (A50-18 strain [Reference Example]) against SARS-CoV-2 mutant strains.
Fig. 30 shows CPE images during recovery culture after creation of vaccine candidate strains 1 to 7 [Examples].
Fig. 31A shows evaluation of temperature sensitivity of vaccine candidate strains 1 to 7 [Examples].
Fig. 31B shows evaluation of temperature sensitivity of rTs-all strain [Example].
Fig. 32 shows neutralizing antibody inducing abilities of vaccine candidate strains 1, 3, 4, 6, and 7 [Examples] at low-titer and low-dose administration.
Fig. 33 shows the results of infection protection test after low-titer and low-dose administration of the vaccine candidate strain 7 [Example].
Fig. 34 shows neutralizing antibody-inducing abilities of the vaccine candidate strains 2 and 5 [Examples] at high-titer and high-dose administration.
Fig. 35 shows evaluation of growth of the vaccine candidate strain 2 [Example] at each temperature.
Fig. 36 shows the results of durability test of humoral immunity induced by administration of the vaccine candidate strain 2 [Example].
Fig. 37 shows the results of infection protection test (weight changes of infected hamsters) by administration of the vaccine candidate strain 2 [Example].
Fig. 38 shows the results of study on reversion of virulence (presence or absence of CPE) during in vivo passage of the vaccine candidate strain 2 [Example].
Fig. 39 shows the results of study on reversion of virulence (sequences of viral RNA extracted from each nasal wash) during in vivo passage of the vaccine candidate strain 2 [Example].
Fig. 40 shows the results of study on reversion of virulence (weight changes) during in vivo passage of the vaccine candidate strain 2 [Example].
Fig. 41 shows evaluation of tissue damages (nasal cavity level 1) by the vaccine candidate strain 2 [Example].
Fig. 42 shows evaluation of tissue damages (nasal cavity level 2) by the vaccine candidate strain 2 [Example].
Fig. 43 shows evaluation of tissue damages (nasal cavity level 3) by the vaccine candidate strain 2 [Example].
Fig. 44 shows evaluation of tissue damages (lung) by the vaccine candidate strain 2 [Example].
Fig. 45 shows neutralizing antibody titers induced by administration of the vaccine candidate strain 2 [Example].
Fig. 46 shows the results of infection protection test (weight changes of infected hamsters) by administration of the vaccine candidate strain 2 [Example].
Fig. 47 shows neutralizing antibody titers induced by administration of the vaccine candidate strain 2 [Example].

### EMBODIMENTS TO CARRY OUT THE INVENTION

### 1. Betacoronavirus attenuated strain

The betacoronavirus attenuated strain of the present invention is characterized by being a betacoronavirus having, non-structural protein(s) having prescribed substitution mutation(s) related to temperature sensitivity, in combination with structural protein(s), accessory protein(s), and/or non-structural protein(s) having prescribed deletion mutation(s) related to growth reduction or other attenuation, as prescribed mutations related to attenuation. Hereinafter, a prescribed substitution mutation related to temperature sensitivity is also referred to as a "temperature-sensitive mutation", a prescribed deletion mutation related to growth reduction is also described as a "growth reducing mutation", and a prescribed deletion mutation other than the growth reducing mutations is also described as a "other attenuating mutation".

In the present invention, the "attenuation" refers to a characteristic of attenuating pathogenicity of a virus against host. In addition, in the present invention, the "temperature sensitivity" refers to a characteristic in which growth at a human body temperature (so-called a lower respiratory tract temperature) is limited and a characteristic having a growth capability specifically at a low temperature (typically, not higher than a human upper respiratory tract temperature). Moreover, in the present invention, the "growth reducing" refers to a characteristic in which the growth is limited and the characteristic is not temperature-specific.

The betacoronavirus attenuated strain of the present invention not only exhibits efficacy as a vaccine by having the prescribed mutation(s) related to attenuation described above, but also has a combination of the substitution mutation(s) and deletion mutation(s) that is less likely to revert to mutation, so that the possibility of causing reversion of virulence is extremely low. In this respect, the usefulness in the case of assuming application to humans is remarkably increased.

The coronavirus is morphologically spherical with a diameter of about 100 to 200 nm and has protrusions on the surface. The coronavirus is virologically classified into Nidovirales, Coronavirinae, Coronaviridae. There is a genome of positive-stranded single-stranded RNA wound around a nucleocapsid protein (hereinafter, also referred to as a "nucleocapsid" or "Nucleocapsid") in the envelope of the lipid bilayer membrane, and a spike protein (hereinafter also referred to as a "spike" or "Spike"), an envelope protein (hereinafter also referred to as an "envelope" or "Envelope"), and a membrane protein (hereinafter also referred to as a "membrane" or "Membrane") are arranged on the surface of the envelope. The size of the viral genome is about 30 kb, the longest among RNA viruses. The nucleocapsid, spike, envelope, and membrane are structural proteins of coronaviruses. NSP1 to NSP16 are non-structural proteins of coronavirus. In addition, ORF7a, ORF7b, ORF8, and the like are accessory proteins of coronavirus. The accessory protein can also be referred to as an accessory protein.

Coronaviruses are classified into groups of alpha, beta, gamma, and delta from genetic characteristics. As coronaviruses infecting humans, there are known four types of human coronaviruses 229E, OC43, NL63, and HKU-1 as causative viruses of cold, and severe acute respiratory syndrome (SARS) coronavirus that occurred in 2002 and Middle East respiratory syndrome (MERS) coronavirus that occurred in 2012, both of which cause serious pneumonia. Human coronaviruses 229E and NL63 are classified into Alphacoronavirus genus, and human coronaviruses OC43, HKU-1, SARS coronavirus, and MERS coronavirus are classified into the Betacoronavirus genus.

SARS-CoV-2 classified as SARS coronavirus has been isolated and identified as a causative virus of the novel coronavirus infection that occurred in Wuhan in 2019. SARS-CoV-2 has been mutated repeatedly from the early Wuhan strain, and mutant strains such as a strain detected in the United Kingdom, a strain detected in South Africa, and a strain detected in India have been found. There are also possibilities that there is a mutant strain that has not yet been detected and that a new mutant strain will occur in the future. In the present invention, the virus included in the genus Betacoronavirus is not limited to the strain of SARS-CoV-2 described above, and includes all other betacoronaviruses (for example, other SARS-CoV-2 mutant strains that will be newly detected in the future and betacoronaviruses other than SARS-CoV-2, and recombinant viruses in which the spike protein of SARS-CoV-2 or betacoronavirus other than SARS-CoV-2 is replaced with a spike protein of at least one of other SARS-CoV-2 and betacoronavirus other than SARS-CoV-2 (including viruses that will be newly detected in the future), and the like).

The prescribed mutations related to attenuation possessed by the betacoronavirus attenuated strain of the present invention will be described based on Table 1 below. Mutation (b), a combination of mutation (e) and mutation (f), and/or mutation (h) indicated as "Temperature-sensitive mutation" in Table 1 are substitution mutations and are responsible mutations that contribute to providing a temperature-sensitive capability essentially held by the betacoronavirus attenuated strain of the present invention. In other words, in the present invention, examples of the temperature-sensitive mutation include three types: "mutation (b)", "combination of mutation (e) and mutation (f)", and "mutation (h)". Typical betacoronavirus attenuated strains of the present invention have one or two types of these three types of temperature-sensitive mutations. Mutation (n), mutation (o) and/or mutation (r) indicated as "Growth reducing mutation" and "Other attenuating mutation" in Table 1 are deletion mutations, and are mutations essentially held by the betacoronavirus attenuated strain of the present invention that are considered to contribute to providing growth reduction or other attenuation (in particular, it is considered that the mutation (r) contributes to providing growth reduction), and expresses excellent attenuation in combination with temperature-sensitive mutation(s). Mutations (a), (c), (d), (g), (i) to (m), (p), and (q) indicated as "Other mutation" in Table 1 are mutations that can be optionally held by the betacoronavirus attenuated strains of the present invention, and the betacoronavirus attenuated strains of the present invention may or may not hold at least any of the other mutations.

**[Table 1]**

| | Mutation sign | Polypeptide | Amino acid before mutation | Amino acid after mutation | In case of mutant strain of SARS-CoV-2 of NC 045512 (NCBI) | | |
|---|---|---|---|---|---|---|---|
| | | | | | SEQ ID NO | Mutation sign in left SEQ ID NO | Mutation position in left SEQ ID NO |
| Other mutation | (a) | NSP3 | Valine (V) | Alanine (A) | 1 | (a') | 404 |
| Temperature-sensitive mutation | (b) | NSP3 | Leucine (L) | Phenylalanine (F) | 1 | (b') | 445 |
| Other mutation | (c) | NSP3 | Lysine (K) | Arginine (R) | 1 | (c') | 1792 |
| Other mutation | (d) | NSP3 | Aspartic acid (D) | Asparagine (N) | 1 | (d') | 1832 |
| Temperature-sensitive mutation | (e) | NSP14 | Glycine (G) | Valine (V) | 2 | (e') | 248 |
| | (f) | NSP14 | Glycine (G) | Serine (S) | 2 | (f') | 416 |
| Other mutation | (g) | NSP14 | Alanine (A) | Valine (V) | 2 | (g') | 504 |
| Temperature-sensitive mutation | (h) | NSP16 | Valine (V) | Isoleucine (I) | 3 | (h') | 67 |
| Other mutation | (i) | Spike | Leucine (L) | Tryptophan (W) | 4 | (i') | 54 |
| Other mutation | (j) | Spike | Threonine (T) | Lysine (K) | 4 | (j') | 739 |
| Other mutation | (k) | Spike | Alanine (A) | Valine (V) | 4 | (k') | 879 |
| Other mutation | (l) | Envelope | Leucine (L) | Proline (P) | 5 | (I') | 28 |
| Other mutation | (m) | Nucleocapsid | Serine (S) | Phenylalanine (F) | 6 | (m') | 2 |
| Other attenuating mutation | (n) | ORF8 | (Complete function) | (Loss-of-function) | 7 | (n') | 1-703 (Deletion)* |
| Other attenuating mutation | (o) | Spike | (Complete) | (Partial deletion) | 4 | (o') | 681-684 (Deletion) |
| Other mutation | (p) | Spike | (Complete) | (Partial deletion) | 4 | (p') | 679-680 (Deletion) 685-686 (Deletion) |
| Other mutation | (q) | Spike | Valine (V) | Isoleucine (I) | 4 | (q') | 687 |
| Growth reducing mutation | (r) | NSP1 | (Complete) | (Partial deletion) | 8 | (r') | 32-39 (Deletion) |

| | | | | | | | |
|---|---|---|---|---|---|---|---|
| The mutation of (b), the combination of mutations of (e) and (f), the mutation of (h), the mutation of (n), the mutation of (o), and the mutation of (r) above are prescribed mutations related to attenuation. *In a case where a sequence corresponding to positions 1 to 703 of SEQ ID NO: 7 is deleted, an amino acid sequence encoded by a base sequence from the middle of ORF7a to the middle of ORF8 is deleted. | | | | | | | |

That is, the essential mutations related to attenuation possessed by the betacoronavirus of the present invention are the following mutation of (b), a combination of the following mutations of (e) and (f), and/or the following mutation of (h), which are temperature-sensitive mutations; and the following mutations of (n), (o) and/or (r), which are growth reducing or other attenuating mutations.
(b) a mutation of an amino acid residue corresponding to leucine at the 445th position of the amino acid sequence set forth in SEQ ID NO: 1 in NSP3,
(e) a mutation of an amino acid residue corresponding to glycine at the 248th position of the amino acid sequence set forth in SEQ ID NO: 2 in NSP14,
(f) a mutation of an amino acid residue corresponding to glycine at the 416th position of the amino acid sequence set forth in SEQ ID NO: 2, in NSP14,
(h) a mutation of an amino acid residue corresponding to valine at the 67th position of the amino acid sequence set forth in SEQ ID NO: 3 in NSP16,
(n) a loss-of-function mutation in ORF8,
(o) a deletion of amino acid residues corresponding to the 681st to 684th positions of the amino acid sequence set forth in SEQ ID NO: 4 in a spike, and
(r) a deletion of an amino acid sequence corresponding to the 32nd to 39th positions of the amino acid sequence set forth in SEQ ID NO: 8 in NSP1.

The above mutation of (n) is acceptable as long as it is a mutation by which the function of ORF8 is lost, but preferably includes a deletion of an amino acid sequence corresponding to the amino acid sequence encoded by the base sequence set forth in SEQ ID NO: 7.

In a preferred embodiment of the present invention, from the viewpoint of controlling the level of temperature sensitivity to the preferred degree, one to two types of mutations or combinations of mutations are selected from the above temperature-sensitive mutations (that is, three types of mutations or combinations of mutations: the mutation of (b), the combination of mutations of (e) and (f), and the mutation of (h)).

In a preferred embodiment of the present invention, among the above growth reducing or other attenuating mutations, the mutation of (o) and the mutation of (r) are preferable.

In a preferred embodiment of the present invention, four types of mutations or combinations of mutations are selected from six types of the prescribed mutations related to attenuation (that is, six types: the mutation of (b), the combination of mutations of (e) and (f), the mutation of (h), the mutation of (n), the mutation of (o), and the mutation of (r)) from the viewpoint of exhibiting preferred immunogenicity as well as preferred attenuation.

In addition to the above essential mutations, the betacoronavirus attenuated strain of the present invention can further hold at least any one of the following mutations (a), (c), (d), (g), (i) to (m), (p), and (q) as other mutation(s).
(a) a mutation of an amino acid residue corresponding to valine at the 404th position of the amino acid sequence set forth in SEQ ID NO: 1 in NSP3,
(c) a mutation of an amino acid residue corresponding to lysine at the 1792nd position of the amino acid sequence set forth in SEQ ID NO: 1 in NSP3,
(d) a mutation of an amino acid residue corresponding to aspartic acid at the 1832nd position of the amino acid sequence set forth in SEQ ID NO: 1 in NSP3,
(g) a mutation of an amino acid residue corresponding to alanine at the 504th position of the amino acid sequence set forth in SEQ ID NO: 2 in NSP14,
(i) a mutation of an amino acid residue corresponding to leucine at the 54th position of the amino acid sequence set forth in SEQ ID NO: 4 in a spike,
(j) a mutation of an amino acid residue corresponding to threonine at the 739th position of the amino acid sequence set forth in SEQ ID NO: 4 in Spike,
(k) a mutation of an amino acid residue corresponding to alanine at the 879th position of the amino acid sequence set forth in SEQ ID NO: 4 in Spike,
(l) a mutation of an amino acid residue corresponding to leucine at the 28th position of the amino acid sequence set forth in SEQ ID NO: 5 in Envelope,
(m) a mutation of an amino acid residue corresponding to serine at the 2nd position of the amino acid sequence set forth in SEQ ID NO: 6 in a nucleocapsid,
(p) a mutation containing deletions of amino acid residues corresponding to the 679th to 680th positions and the 685th to 686th positions of the amino acid sequence set forth in SEQ ID NO: 4 in Spike, and
(q) a mutation of an amino acid residue corresponding to valine at the 687th position of the amino acid sequence set forth in SEQ ID NO: 4 in Spike.

When the betacoronavirus attenuated strain of the present invention has other mutation, it is preferable to have the mutation of (g) among the other mutations from the viewpoint of enhancing temperature sensitivity. When the betacoronavirus attenuated strain of the present invention has the mutation of (g) as other mutation, the mutation of (g) is preferably used together with the combination of the mutations of (e) and (f) from the viewpoint of enhancing temperature sensitivity.

SEQ ID NO: 1 is the amino acid sequence of NSP3 in SARS-CoV-2 of NC_045512 (NCBI); SEQ ID NO: 2 is the amino acid sequence of NSP14 in SARS-CoV-2 of NC_045512 (NCBI); and SEQ ID NO: 3 is the amino acid sequence of NSP16 in SARS-CoV-2 of NC_045512 (NCBI).

In addition, SEQ ID NO: 4 is the amino acid sequence of the spike in SARS-CoV-2 of NC_045512 (NCBI); SEQ ID NO: 5 is the amino acid sequence of the envelope in SARS-CoV-2 of NC_045512 (NCBI); and SEQ ID NO: 6 is the amino acid sequence of the nucleocapsid in SARS-CoV-2 of NC_045512 (NCBI).

Further, SEQ ID NO: 7 is the base sequence of the part of open reading frames of SARS-CoV-2 of NC_045512 (NCBI), specifically, across a portion of ORF7a, the entire ORF7b, and most of ORF8; and SEQ ID NO: 8 is the amino acid sequence of NSP1 in SARS-CoV-2 of NC_045512 (NCBI).

The "amino acid residue corresponding" refers to an amino acid residue present at the above prescribed position in the amino acid sequences of SEQ ID NOs: 1 to 4 (or 1 to 6), 8, or the amino acid sequence encoded by the base sequence set forth in SEQ ID NO: 7 when the betacoronavirus attenuated strain of the present invention is a mutant strain of SARS-CoV-2 of NC_045512 (NCBI), and refers to an amino acid residue present at a position corresponding to the above prescribed position in the amino acid sequences of SEQ ID NOs: 1 to 4 (or 1 to 6), 8 of the polypeptide possessed by another betacoronavirus, or the amino acid sequence encoded by the base sequence set forth in SEQ ID NO: 7 when the betacoronavirus attenuated strain of the present invention is another betacoronavirus mutant strain other than the above mutant strain. The corresponding position can be identified by aligning amino acid sequences between proteins having the amino acid sequences of SEQ ID NOs: 1 to 4 (or 1 to 6) and 8 or proteins having the amino acid sequence encoded by the base sequence set forth in SEQ ID NO: 7 of SARS-CoV-2 of NC_045512 (NCBI) and proteins of other betacoronaviruses corresponding to the proteins.

The virus attenuated strain of the present invention is not limited to a mutant strain of specific SARS-CoV-2 listed in NC_045512 (NCBI) as long as an amino acid residue or amino acid sequence corresponding to the above prescribed positions in the amino acid sequences of SEQ ID NOs: 1 to 4 (or 1 to 6), 8, or the amino acid sequence encoded by the base sequence set forth in SEQ ID NO: 7 is mutated, but includes other betacoronavirus mutant strains [i.e., any other mutant strains of SARS-CoV-2 and mutant strains of viruses other than SARS-CoV-2 which are included in the Betacoronavirus genus]. The mutant strains of specific SARS-CoV-2 listed in NC_045512 (NCBI) are defined as mutant strains in which at least any one of amino acid residues or amino acid sequences at the above specific positions in the amino acid sequences of SEQ ID NOs: 1 to 4 (or 1 to 6), 8, or the amino acid sequence encoded by the base sequence set forth in SEQ ID NO: 7 in the specific SARS-CoV-2 is mutated, and the other betacoronavirus mutant strains refer to both any other mutant strains of SARS-CoV-2 [i.e., mutant strains in which amino acid residues or amino acid sequences corresponding to the above prescribed positions in the amino acid sequences of SEQ ID NOs: 1 to 4 (or 1 to 6), 8, or the amino acid sequence encoded by the base sequence set forth in SEQ ID NO: 7 in any other SARS-CoV-2 are mutated] and mutant strains of viruses other than SARS-CoV-2 which are included in the Betacoronavirus genus [i.e., mutant strains in which amino acid residues or amino acid sequences corresponding to the above prescribed positions in the amino acid sequences of SEQ ID NOs: 1 to 4 (or 1 to 6), 8, or the amino acid sequence encoded by the base sequence set forth in SEQ ID NO: 7 in viruses other than SARS-CoV-2 which are included in the Betacoronavirus genus are mutated]. Any other mutant strains of SARS-CoV-2 and mutant strains of viruses other than SARS-CoV-2 which are included in the Betacoronavirus genus also include mutant strains of recombinant viruses in which the spike protein of SARS-CoV-2 or betacoronavirus other than SARS-CoV-2 is replaced with a spike protein of at least one of other SARS-CoV-2 and betacoronaviruses other than SARS-CoV-2 (including viruses that will be newly detected in the future).

Each of the amino acid sequences of SEQ ID NOs: 1 to 4 (or 1 to 6), 8, or the sequence corresponding to the base sequence set forth in SEQ ID NO: 7 in other betacoronavirus mutant strains is allowed to differ from the amino acid sequences of SEQ ID NOs: 1 to 4 (or 1 to 6), 8, or the base sequence set forth in SEQ ID NO: 7, as long as it does not significantly affect the characteristics of the polypeptide. The phrase "does not significantly affect the characteristics of the polypeptide" refers to a state in which a function as a non-structural protein, a structural protein, or an accessory protein of each polypeptide is maintained. Specifically, at a site other than an amino acid or base sequence corresponding to the prescribed mutations related to attenuation in the amino acid sequences of SEQ ID NOs: 1 to 4, 8, or the base sequence set forth in SEQ ID NO: 7, or in the case of further having other mutations, at site(s) other than amino acid residue(s) corresponding to the other mutations in SEQ ID NOs: 5 and 6 described above (hereinafter, a site other than amino acid residues or bases corresponding to these mutations is also referred to as an "any different site"), a difference from SEQ ID NOs: 1 to 4, 7, 8 (or 1 to 8) is acceptable. The acceptable difference may be one type of difference selected from substitution, addition, insertion, and deletion (e.g., substitution), or may include two or more types of differences (e.g., substitution and insertion). A sequence identity calculated by comparing only any different sites of amino acid sequences corresponding to SEQ ID NOs: 1 to 4 (or 1 to 6), 8 or the base sequence set forth in SEQ ID NO: 7 in any other SARS-CoV-2 and the amino acid sequences set forth in SEQ ID NOs: 1 to 4 (or 1 to 6), 8 or the base sequence set forth in SEQ ID NO: 7 may be not less than 50%. In any other SARS-CoV-2, the sequence identity is preferably not less than 60% or not less than 70%, more preferably not less than 80%, further preferably not less than 85% or not less than 90%, still more preferably not less than 95%, not less than 96%, not less than 97%, or not less than 98%, still more preferably not less than 99%, and particularly preferably not less than 99.3%, not less than 99.5%, not less than 99.7%, or not less than 99.9%. In any remaining betacoronavirus, the sequence identity is preferably not less than 60%. Here, the "sequence identity" shows an identity value of an amino acid sequence obtained by BLAST PACKAGE [sgi32 bit edition, Version 2.0.12; available from National Center for Biotechnology Information (NCBI)] bl2seq program (Tatiana A. Tatsusova, Thomas L. Madden, FEMS Microbiol. Lett., Vol. 174, p247-250, 1999). Parameters may be set to Gap insertion Cost value: 11 and Gap extension Cost value: 1.

That is, the betacoronavirus attenuated strain of the present invention is more specifically as follows:
A betacoronavirus attenuated strain containing non-structural protein(s), accessory protein(s), and structural protein(s) consisting of at least any one of the following polypeptides (I), (II) and (III):
(I) at least any one of the following polypeptides (I-1) to (I-3) and at least any one of the following polypeptides (1-4) to (I-6):
   (I-1) a polypeptide (NSP3) consisting of an amino acid sequence having, as a temperature-sensitive mutation, a substitution mutation of leucine at the 445th position (b') in the amino acid sequence set forth in SEQ ID NO: 1,
   (I-2) a polypeptide (NSP14) consisting of an amino acid sequence having, as temperature-sensitive mutations, a substitution mutation of glycine at the 248th position (e') and a substitution mutation of glycine at the 416th position (f') in the amino acid sequence set forth in SEQ ID NO: 2,
   (I-3) a polypeptide (NSP16) consisting of an amino acid sequence having, as a temperature-sensitive mutation, a substitution mutation of valine at the 67th position (h') in the amino acid sequence set forth in SEQ ID NO: 3,
   (I-4) a polypeptide (ORF) consisting of an amino acid sequence having, as other attenuating mutation, a deletion mutation (n') of the amino acid sequence encoded by the base sequence set forth in SEQ ID NO: 7,
   (I-5) a polypeptide (spike) consisting of an amino acid sequence having, as other attenuating mutation, a deletion mutation (o') at the 681st to 684th positions in the amino acid sequence set forth in SEQ ID NO: 4, and
   (I-6) a polypeptide (NSP1) consisting of an amino acid sequence having, as a growth reducing mutation, a deletion mutation (r') at the 32nd to 39th positions in the amino acid sequence set forth in SEQ ID NO: 8;
(II) a polypeptide in which in the amino acid sequence of the polypeptide (I), one or more amino acid residues other than the amino acid residue(s) related to the temperature-sensitive mutation(s) and the amino acid sequence(s) related to the growth reducing or other attenuating mutation(s) are substituted, added, inserted, or deleted, and the polypeptide constituting a betacoronavirus that has acquired a temperature-sensitive capability and an attenuation capability; and
(III) a polypeptide having a sequence identity of not less than 50% in the amino acid sequence of the polypeptide (I) excluding the amino acid residue(s) related to the temperature-sensitive mutation(s) and the amino acid sequence(s) related to the growth reducing or other attenuating mutation(s), and the polypeptide constituting a betacoronavirus that has acquired a temperature-sensitive capability and an attenuation capability.

When the more specific betacoronavirus attenuated strain described above contains other mutation(s) in addition to the temperature-sensitive mutation(s) and growth reducing or other attenuating mutation(s), as shown below, the polypeptides (I-1) and (I-2) described above (non-structural proteins) and the polypeptide (I-5) described above (structural protein) may be the polypeptides (I-1a) and (I-2a) and (I-5a) described below that also have other mutation(s) in addition to temperature-sensitive mutation(s) and growth reducing or other attenuating mutation(s), respectively, and the polypeptide (I) described above may further contain the polypeptides (I-7a) and (I-8a) described below (structural proteins) that have other mutation(s). A betacoronavirus attenuated strain containing structural protein(s), accessory protein(s) and non-structural protein(s) consisting of at least any one of the following polypeptides (I), (II), and (III):
(I) at least any one of the following polypeptides (I-1a), (I-2a), and (I-3) ; and at least any one of the following polypeptides (I-4), (I-5a), and (I-6), or in addition thereto, at least one of the following polypeptides (I-7a) and (I-8a):
   (I-1a) a polypeptide (NSP3) consisting of an amino acid sequence having, as a temperature-sensitive mutation, a mutation of leucine at the 445th position (b') and, as other mutation(s), a mutation of at least any one of a mutation of valine at the 404th position (a'), a mutation of lysine at the 1792nd position (c'), and a mutation of aspartic acid at the 1832nd position (d') in the amino acid sequence set forth in SEQ ID NO: 1,
   (I-2a) a polypeptide (NSP14) consisting of an amino acid sequence having, as temperature-sensitive mutations, a mutation of glycine at the 248th position (e') and a mutation of glycine at the 416th position (f) and, as other mutation, a mutation of alanine at the 504th position (g') in the amino acid sequence set forth in SEQ ID NO: 2,
   (I-3) a polypeptide (NSP16) consisting of an amino acid sequence having, as a temperature-sensitive mutation, a mutation of valine at the 67th position (h') in the amino acid sequence set forth in SEQ ID NO: 3,
   (I-4) a polypeptide (ORF) consisting of an amino acid sequence having, as other attenuating mutation, a deletion mutation (n') of the amino acid sequence encoded by the base sequence set forth in SEQ ID NO: 7,
   (I-5a) a polypeptide (spike) consisting of an amino acid sequence having, as other attenuating mutation, a deletion mutation at the 681st to 684th positions (o'); and, as other mutation(s), a mutation of at least any one of a mutation of leucine at the 54th position (i'), a mutation of threonine at the 739th position (j'), and a mutation of alanine at the 879th position (k'), and a mutation of at least one of a deletion mutation of amino acid sequences at the 679th to 680th positions and the 685th to 686th positions (p') and a mutation of valine at the 687th position (q') in the amino acid sequence set forth in SEQ ID NO: 4,
   (I-6) a polypeptide (NSP1) consisting of an amino acid sequence having, as a growth reducing mutation, a deletion mutation (r') at the 32nd to 39th positions in the amino acid sequence set forth in SEQ ID NO: 8
   (I-7a) a polypeptide (envelope) consisting of an amino acid sequence having, as other mutation, a mutation of leucine at the 28th position (l') in the amino acid sequence set forth in SEQ ID NO: 5,
   (I-8a) a polypeptide (nucleocapsid) consisting of an amino acid sequence having, as other mutation, a mutation of serine at the 2nd position (m') in the amino acid sequence set forth in SEQ ID NO: 6;
(II) a polypeptide in which in the amino acid sequence of the polypeptide (I), one or more amino acid residues other than the amino acid residue(s) related to the temperature-sensitive mutation(s), the growth reducing or other attenuating mutation(s) and the amino acid residue(s) or amino acid sequence(s) related to the other mutation(s) are substituted, added, inserted, or deleted, and the polypeptide constituting a betacoronavirus that has acquired a temperature-sensitive capability and an attenuation capability; and
(III) a polypeptide having a sequence identity of not less than 50% in the amino acid sequence of the polypeptide (I) excluding the amino acid residue(s) related to the temperature-sensitive mutation(s), the growth reducing or other attenuating mutation(s) and the amino acid residue(s) or amino acid sequence(s) related to the other mutation(s), and constituting a betacoronavirus that has acquired a temperature-sensitive capability and an attenuation capability.

The above mutations of (a') to (r') refer to mutations when the mutations of (a) to (r) are specifically present in the amino acid sequences of SEQ ID NOs: 1 to 6, the base sequence of SEQ ID NO: 7, and the amino acid sequence of SEQ ID NO: 8, respectively. In other words, the above polypeptide (I) is a polypeptide obtained by introducing temperature-sensitive mutation(s), growth reducing or other attenuating mutation(s), or in addition thereto, other mutation(s) into a polypeptide consisting of the amino acid sequences of SEQ ID NOs: 1 to 6, the amino acid sequence encoded by the base sequence of SEQ ID NO: 7, and the amino acid sequence of SEQ ID NO: 8 possessed by SARS-CoV-2 of NC_045512 (NCBI). In addition, the above polypeptides (II) and (III) are obtained by introducing temperature-sensitive mutation(s), growth reducing or other attenuating mutation(s), or in addition thereto, other mutation(s) into a polypeptide consisting of amino acid sequences corresponding to the amino acid sequences of SEQ ID NOs: 1 to 6, the amino acid sequence encoded by the base sequence of SEQ ID NO: 7, and the amino acid sequence of SEQ ID NO: 8, which are possessed by another betacoronavirus. Preferred ranges of the sequence identity of the above polypeptides (II) and (III) are as described above.

The betacoronavirus can acquire temperature sensitivity by having the above temperature-sensitive mutation, and can acquire excellent attenuation by having the above growth reducing or other attenuating mutation together with the above temperature-sensitive mutation. In the virus attenuated strain of the present invention, a growth capability at a human lower respiratory tract temperature is at least decreased as compared with a growth capability at a temperature lower than a human lower respiratory tract temperature, and preferably, the virus attenuated strain of the present invention does not have a growth capability at a human lower respiratory tract temperature. In the present invention, the temperature-sensitive capability can be confirmed by the fact that after Vero cells are infected with the virus attenuated strain at MOI = 0.01 at a human lower respiratory tract temperature and then the virus attenuated strain is cultured at a human lower respiratory tract temperature for 1 day a virus titer (TCID50/mL) in culture supernatant is decreased, for example, by not less than 10², preferably by not less than 10³, as compared with a virus titer in culture supernatant after Vero cells are infected with the virus attenuated strain at MOI = 0.01 at a human upper respiratory tract temperature and then the virus attenuated strain is cultured at a human upper respiratory tract temperature for 1 day.

Typically, in the virus attenuated strain of the present invention, a growth capability at a human lower respiratory tract temperature is decreased as compared with a growth capability at a human lower respiratory tract temperature in the case of not having the above temperature-sensitive mutation. This can be confirmed by the fact that, after Vero cells are infected with the virus attenuated strain at MOI = 0.01 at a human lower respiratory tract temperature and then the virus attenuated strain is cultured at a human lower respiratory tract temperature for 1 day, a virus titer (TCID50/mL) in culture supernatant is decreased, for example, by not less than 10², preferably by not less than 10³, as compared with a virus titer in culture supernatant after Vero cells are infected with a strain not having the above temperature-sensitive mutation at MOI = 0.01 at a human lower respiratory tract temperature and then the strain is cultured at a human lower respiratory tract temperature for 1 day.

Representative examples of the human lower respiratory tract temperature include about 37°C, and specifically include a temperature higher than the upper respiratory tract temperature described below, preferably 36 to 38°C, and more preferably 36.5 to 37.5°C or 37 to 38°C. In addition, the virus attenuated strain of the present invention may have a growth capability at a temperature lower than a human lower respiratory tract temperature. For example, the temperature lower than the human lower respiratory tract temperature may include, for example, a human upper respiratory tract temperature (as a specific example, about 32°C to 35.5°C).

The above temperature-sensitive mutations are not present on receptor-binding domains of a spike protein present on a surface of the virus, which is important when the virus infects cells. Therefore, it is reasonably expected that not only the SARS-CoV-2 listed in NC_045512 (NCBI) but also other betacoronaviruses can be made temperature-sensitive by introducing the above temperature-sensitive mutation. In other words, it is reasonably expected that, even if a mutation occurs so as to alter the immunogenicity of the virus due to worldwide infection, temperature sensitivity can be provided for the mutant virus by further introducing the above temperature-sensitive mutation into the mutant virus.

Regarding the temperature-sensitive mutations, the above mutation of (b) may be a substitution with an amino acid residue other than leucine, the above mutation of (e) may be a substitution with an amino acid residue other than glycine, the above mutation of (f) may be a substitution with an amino acid residue other than glycine, and the above mutation of (h) may be a substitution with an amino acid residue other than valine. Regarding other mutations, the above mutation of (a) may be a substitution with an amino acid residue other than valine, the above mutation of (c) may be a substitution with an amino acid residue other than lysine, the above mutation of (d) may be a substitution with an amino acid residue other than aspartic acid, the above mutation of (g) may be a substitution with an amino acid residue other than alanine, the above mutation of (i) may be a substitution with an amino acid residue other than leucine, the above mutation of (j) may be a substitution with an amino acid residue other than threonine, the above mutation of (k) may be a substitution with an amino acid residue other than alanine, the above mutation of (l) may be a substitution with an amino acid residue other than leucine, the above mutation of (m) may be a substitution with an amino acid residue other than serine, and the above mutation of (q) may be a substitution with an amino acid residue other than valine.

In a preferred example of the virus attenuated strain of the present invention, regarding the temperature-sensitive mutations, the mutation of (b) is a substitution with phenylalanine, the mutation of (e) is a substitution with valine and the mutation of (f) is a substitution with serine, and/or the mutation of (h) is a substitution with isoleucine. When the preferred example further has other mutations, regarding those mutations, the mutation of (a) is a substitution with alanine, the mutation of (c) is a substitution with arginine, the mutation of (d) is a substitution with asparagine, the mutation of (g) is a substitution with valine, the mutation of (i) is a substitution with tryptophan, the mutation of (j) is a substitution with lysine, the mutation of (k) is a substitution with valine, the mutation of (l) is a substitution with proline, the mutation of (m) is a substitution with phenylalanine, and/or the mutation (q) is a substitution with isoleucine.

In another example of the virus attenuated strain of the present invention, the substitution may be a so-called conservative substitution. The conservative substitution refers to a substitution with an amino acid having a similar structure and/or characteristic, and examples of the conservative substitution include a substitution with another non-polar amino acid if the amino acid before substitution is a non-polar amino acid, a substitution with another non-charged amino acid if the amino acid before substitution is a non-charged amino acid, a substitution with another acidic amino acid if the amino acid before substitution is an acidic amino acid, and a substitution with another basic amino acid if the amino acid before substitution is a basic amino acid. In general, the "non-polar amino acid" includes alanine, valine, leucine, isoleucine, proline, methionine, phenylalanine, and tryptophan, the "non-charged amino acid" includes glycine, serine, threonine, cysteine, tyrosine, asparagine, and glutamine, the "acidic amino acid" includes aspartic acid and glutamic acid, and the "basic amino acid" includes lysine, arginine, and histidine.

A more preferred example of the virus attenuated strain of the present invention includes a mutant strain of the SARS-CoV-2 listed in NC_045512 (NCBI), in which the mutation of (b) (i.e., the mutation of (b')) is a substitution of leucine at the 445th position of the amino acid sequence set forth in SEQ ID NO: 1 with phenylalanine in NSP3 (L445F); the mutation of (e) (i.e., the mutation of (e')) is a substitution of glycine at the 248th position of the amino acid sequence set forth in SEQ ID NO: 2 with valine in NSP14 (G248V), and the mutation of (f) (i.e., the mutation of (f)) is a substitution of glycine at the 416th position of the amino acid sequence set forth in SEQ ID NO: 2 with serine in NSP14 (G416S); and/or the mutation of (h) (i.e., the mutation of (h')) is a substitution of valine at the 67th position of the amino acid sequence set forth in SEQ ID NO: 3 with isoleucine in NSP16 (V67I). An example when the more preferred example also has other mutation(s) includes the mutant strains, in which the mutation of (a) (i.e., the mutation of (a')) is the substitution of valine at the 404th position of the amino acid sequence set forth in SEQ ID NO: 1 with alanine in NSP3 (V404A); the mutation of (c) (i.e., the mutation of (c')) is a substitution of lysine at the 1792nd position in the amino acid sequence set forth in SEQ ID NO: 1 with arginine in NSP3 (K1792R); the mutation of (d) (i.e., the mutation of (d')) is a substitution of aspartic acid at the 1832nd position of the amino acid sequence set forth in SEQ ID NO: 1 with asparagine in NSP3 (D1832N); the mutation of (g) (i.e., the mutation of (g')) is a substitution of alanine at the 504th position of the amino acid sequence set forth in SEQ ID NO: 2 with valine in NSP14 (A504V); the mutation of (i) (i.e., the mutation of (i')) is a substitution of leucine at the 54th position of the amino acid sequence set forth in SEQ ID NO: 4 with tryptophan in Spike (L54W); the mutation of (j) (i.e., the mutation of (j')) is a substitution of threonine at the 739th position of the amino acid sequence set forth in SEQ ID NO: 4 with lysine in Spike (T739K); the mutation of (k) (i.e., the mutation of (k')) is a substitution of alanine at the 879th position of the amino acid sequence set forth in SEQ ID NO: 4 with valine in Spike (A879V); the mutation of (l) (i.e., the mutation of (l')) is a substitution of leucine at the 28th position of the amino acid sequence set forth in SEQ ID NO: 5 with proline in Envelope (L28P); the mutation of (m) (i.e., the mutation of (m')) is a substitution of serine at the 2nd position of the amino acid sequence set forth in SEQ ID NO: 6 with phenylalanine in a nucleocapsid (S2F); and/or the mutation of (q) (i.e., the mutation of (q')) is a substitution of valine at the 687th position of the amino acid sequence set forth in SEQ ID NO: 4 with isoleucine in Spike (V687I).

Particularly preferred examples of the virus attenuated strain of the present invention include the following strains:
- A strain having, as temperature-sensitive mutations, the mutation of (e) (preferably the mutation of (e') and/or G248V) and the mutation of (f) (preferably the mutation of (f') and/or G416S), and the mutation of (h) (preferably the mutation of (h') and/or V67I); and, as growth reducing or other attenuating mutations, the mutation of (n) (preferably the mutation of (n')), the mutation of (o), and the mutation of (r); or a strain further having, as other mutations, the mutation of (g) (preferably the mutation of (g') and/or A504V), the mutation of (p), and the mutation of (q) (preferably the mutation of (q') and/or V687I)
- A strain having, as temperature-sensitive mutations, the mutation of (e) (preferably the mutation of (e') and/or G248V) and the mutation of (f) (preferably the mutation of (f') and/or G416S); and, as growth reducing or other attenuating mutations, the mutation of (n) (preferably the mutation of (n')), the mutation of (o), and the mutation of (r); or a strain further having, as other mutations, the mutation of (g) (preferably the mutation of (g') and/or A504V), the mutation of (p), and the mutation of (q) (preferably the mutation of (q') and/or V687I)
- A strain having, as temperature-sensitive mutations, the mutation of (e) (preferably the mutation of (e') and/or G248V), the mutation of (f) (preferably the mutation of (f') and/or G416S), and the mutation of (h) (preferably the mutation of (h') and/or V67I); and, as other attenuating mutations, the mutation of (n) (preferably the mutation of (n')), and the mutation of (o); or a strain further having, as other mutations, the mutation of (g) (preferably the mutation of (g') and/or A504V), the mutation of (p), and the mutation of (q) (preferably the mutation of (q') and/or V687I)
- A strain having, as temperature-sensitive mutations, the mutation of (b) (preferably the mutation of (b') and/or L445F), the mutation of (e) (preferably the mutation of (e') and/or G248V), and the mutation of (f) (preferably the mutation of (f') and/or G416S); and, as other attenuating mutations, the mutation of (n) (preferably the mutation of (n')), and the mutation of (o); or a strain further having, as other mutations, the mutation of (c) (preferably the mutation of (c') and/or K1792R), the mutation of (g) (preferably the mutation of (g') and/or A504V), the mutation of (p), and the mutation of (q) (preferably the mutation of (q') and/or V687I)
- A strain having, as temperature-sensitive mutations, the mutation of (b) (preferably the mutation of (b') and/or L445F), and the mutation of (h) (preferably the mutation of (h') and/or V67I); and, as other attenuating mutations, the mutation of (n) (preferably the mutation of (n')), and the mutation of (o); or a strain further having, as other mutations, the mutation of (c) (preferably the mutation of (c') and/or K1792R), the mutation of (p), and the mutation of (q) (preferably the mutation of (q') and/or V687I)
- A strain having, as temperature-sensitive mutations, the mutation of (e) (preferably the mutation of (e') and/or G248V), the mutation of (f) (preferably the mutation of (f') and/or G416S), and the mutation of (h) (preferably the mutation of (h') and/or V67I); and, as growth reducing or other attenuating mutations, the mutation of (n) (preferably the mutation of (n')), and the mutation of (r) (preferably the mutation of (r')); or a strain further having, as other mutation, the mutation of (g) (preferably the mutation of (g') and/or A504V)

Most preferred examples of the virus attenuated strain of the present invention include the following strain:
A strain having, as temperature-sensitive mutations, the mutation of (e) (preferably the mutation of (e') and/or G248V) and the mutation of (f) (preferably the mutation of (f') and/or G416S); and, as growth reducing or other attenuating mutations, the mutation of (n) (preferably the mutation of (n')), the mutation of (o), and the mutation of (r); and a strain further having, as other mutations, the mutation of (g) (preferably the mutation of (g') and/or A504V), the mutation of (p), and the mutation of (q) (preferably the mutation of (q') and/or V687I)

### 2. Vaccine

### 2-1. Active ingredient of attenuated vaccine

As described above, since the betacoronavirus attenuated strain described in "1. Betacoronavirus attenuated strain" can efficiently grow only at a temperature lower than a human lower respiratory tract temperature by having a temperature-sensitive mutation, it can be expected that it cannot efficiently grow at least in a deep part of a living body, especially in the lower respiratory tract including lungs, which cause serious disorders, so that the pathogenicity is significantly decreased. In addition, the betacoronavirus attenuated strain shows limited growth regardless of temperature by having growth reducing or other attenuating mutations, and has excellent attenuation in combination with the above temperature-sensitive mutations. By taking such a combined form, the betacoronavirus attenuated strain has a characteristic that, when a live attenuated vaccine is accompanied by growth in a host's body, even when the temperature-sensitive mutation is lost, the deletion mutation, which is a growth reducing or other attenuating mutation, is less likely to revert to mutation, and thus it can be expected that the attenuation can be maintained.

Therefore, the virus attenuated strain can be used as a live attenuated vaccine by infecting a living body as an attenuated virus itself. Therefore, the present invention also provides a vaccine containing the above betacoronavirus attenuated strain as an active ingredient. Details of the active ingredient are as described in "1. Betacoronavirus attenuated strain".

### 2-2. Active ingredient of gene vaccine

As described in the above "1. Betacoronavirus attenuated strain", the prescribed mutations contribute to providing attenuation. Therefore, the present invention also provides a betacoronavirus gene vaccine containing, as an active ingredient, a gene encoding non-structural protein(s), accessory protein(s), and structural protein(s) having the prescribed mutation(s) as described in the above. Details of the prescribed mutation(s) contained in the active ingredient are as described in "1. Betacoronavirus attenuated strain".

### 2-3. Targeted viruses

It can be reasonably expected that the vaccine of the present invention is effective against not only the early Wuhan strain of SARS-CoV-2 but also a wide range of SARS-CoV-2 virus-associated strains and viruses included in the Betacoronavirus genus other than SARS-CoV-2, including the variants detected in the United Kingdom in September 2020 and detected in South Africa in October 2020, and other known variants, as well as unknown mutant strains yet to be detected. Therefore, the vaccine of the present invention targets betacoronaviruses.

### 2-4. Other ingredients

The vaccine of the present invention can contain another ingredient such as an adjuvant, a buffer, an isotonizing agent, a soothing agent, a preservative, an antioxidant, a deodorant, a light-absorbing dye, a stabilizer, a carbohydrate, a casein digest, any sort of vitamin or the like, in addition to the above active ingredient, according to the purpose, use, and the like.

Examples of the adjuvant include animal oils (squalene and the like) or hardened oils thereof; vegetable oils (palm oil, castor oil, and the like) or hardened oils thereof; oily adjuvants including anhydrous mannitol/oleic acid ester, liquid paraffin, polybutene, caprylic acid, oleic acid, higher fatty acid ester, and the like; water-soluble adjuvants such as PCPP, saponin, manganese gluconate, calcium gluconate, manganese glycerophosphate, soluble aluminum acetate, aluminum salicylate, acrylic acid copolymer, methacrylic acid copolymer, maleic anhydride copolymer, alkenyl derivative polymer, oil-in-water emulsion, and cationic lipid containing quaternary ammonium salt; precipitating adjuvants such as aluminum salts such as aluminum hydroxide (alum), aluminum phosphate, and aluminum sulfate or combinations thereof, and sodium hydroxide; microorganism-derived toxin components such as cholera toxin and E. coli heat-labile toxin; and other ingredients (bentonite, muramyl dipeptide derivatives, interleukin, and the like).

Examples of the buffer include buffers such as phosphate, acetate, carbonate, and citrate. Examples of the isotonizing agent include sodium chloride, glycerol, D-mannitol, and the like. Examples of the soothing agent include benzyl alcohol and the like. Examples of the preservative include thimerosal, para-hydroxybenzoic acid esters, phenoxyethanol, chlorobutanol, benzyl alcohol, phenethyl alcohol, dehydroacetic acid, sorbic acid, antibiotics, synthetic antibacterial agents, and the like. Examples of the antioxidant include sulfite, ascorbic acid, and the like.

Examples of the light-absorbing dye include riboflavin, adenine, adenosine, and the like. Examples of the stabilizer include chelating agents, reducing agents, and the like. Examples of the carbohydrate include sorbitol, lactose, mannitol, starch, sucrose, glucose, dextran, and the like.

Furthermore, the vaccine of the present invention may contain one or more other vaccines against viruses or bacteria that cause diseases other than betacoronavirus infection, such as COVID-19. In other words, the vaccine of the present invention may be prepared as a combination vaccine containing another vaccine.

### 2-5. Dosage form

A dosage form of the vaccine of the present invention is not particularly limited, and can be appropriately determined based on an administration method, storage conditions, and the like. Specific examples of the dosage form include liquid formulations and solid formulations, and the like, and more specifically, oral administration agents such as tablets, capsules, powders, granules, pills, liquids, and syrups; parenteral administration agents such as dried formulations including freeze-dried formulations, injections, sprays, and patches (concretely, intramuscular administration agents, intradermal administration agents, subcutaneous administration agents, nasal administration agents, transdermal administration agents, and the like).

### 2-6. Administration method

A method for administering the vaccine of the present invention is not particularly limited, and may be any of injection administration such as intramuscular, intraperitoneal, intradermal, and subcutaneous administration; inhalation administration from nasal cavity and oral cavity; oral administration, and the like, but injection administration such as intramuscular, intradermal, and subcutaneous administration (intramuscular administration, intradermal administration, and subcutaneous administration), inhalation administration from nasal cavity (nasal administration), and absorption administration from the skin (transdermal administration) are preferable, and nasal administration is more preferable.

### 2-7. Applicable subject

A subject to which the vaccine of the present invention is applied is not particularly limited as long as the subject that can develop various symptoms by betacoronavirus infection (preferably a subject that can develop COVID-19 symptoms by SARS-CoV-2 infection), and examples thereof include mammals, and more specifically, humans; pet animals such as dogs and cats; and experimental animals such as rats, mice, and hamsters.

### 2-8. Dose

A dose of the vaccine of the present invention is not particularly limited, and can be appropriately determined according to a type of an active ingredient, an administration method, and an applicable subject (conditions such as age, weight, sex, and presence or absence of underlying disease).

In addition, the amount per dose of the vaccine of the present invention for a human is not less than 1 × 10 PFU/body, preferably not less than 1 × 10² PFU/body, more preferably not less than 2 × 10² PFU/body, still more preferably not less than 1 × 10³ PFU/body, and still more preferably not less than 2 × 10³ PFU/body. Moreover, the amount per dose of the vaccine of the present invention for a human is also not more than 6 × 10¹¹ PFU/body, preferably not more than 1 × 10¹¹ PFU/body, more preferably not more than 6 × 10¹⁰ PFU/body, and still more preferably not more than 1 × 10¹⁰ PFU/body.

Further, the amount per dose of the vaccine of the present invention for a human is not less than 1 × 10 TCID50/body, preferably not less than 1 × 10² TCID50/body, more preferably not less than 2 × 10² TCID50/body, more preferably not less than 1 × 10³ TCID50/body, and more preferably not less than 2 × 10³ TCID50/body. Furthermore, the amount per dose of the vaccine of the present invention for a human is also not more than 6 × 10¹¹ TCID50/body, preferably not more than 1 × 10¹¹ TCID50/body, more preferably not more than 6 × 10¹⁰ TCID50/body, and still more preferably not more than 1 × 10¹⁰ TCID50/body.

### 3. Method for producing betacoronavirus attenuated strain

A method for producing the betacoronavirus attenuated strain of the present invention is not particularly limited, and can be appropriately determined by those skilled in the art based on the above amino acid sequence information. For example, from the viewpoint of producing a vaccine that is relatively inexpensive and has little lot difference, the production method preferably includes a reverse genetics method using an artificial chromosome such as a bacterial artificial chromosome (BAC) or a yeast artificial chromosome (YAC), or CPER method or the like using genomic fragments of a betacoronavirus.

In a method for reconstructing the virus by the reverse genetics method, first, a genome of a strain (parent strain) having no temperature-sensitive mutation and no growth reducing or other attenuating mutation of the betacoronavirus attenuated strain is cloned. The parent strain used at this time may be a betacoronavirus, and concretely, it can be selected from the group consisting of the above SARS-CoV-2 listed in NC_045512 (NCBI), the above any other SARS-CoV-2, and viruses other than SARS-CoV-2 which are included in the Betacoronavirus genus.

Furthermore, when an artificial chromosome is used in the reverse genetics method, a full-length DNA of a viral genome is cloned into BAC DNA, YAC DNA, or the like, and a transcription promoter sequence for eukaryotic cells is inserted upstream of a sequence of the virus. Examples of the promoter sequence include CMV promoter and CAG promoter. A ribozyme sequence and a polyA sequence are inserted downstream of a sequence of the virus. Examples of a ribozyme sequence include a hepatitis D virus ribozyme and a hammerhead ribozyme. Examples of the polyA sequence include polyA of Simian 40 virus.

On the other hand, when using the CPER method in the reverse genetics method, full-length DNA of a viral genome is divided into several fragments and cloned. Examples of a method for obtaining fragments include a method for artificial synthesis of a nucleic acid and a PCR method using a plasmid obtained by cloning the above artificial chromosome or fragments as a template.

In order to introduce at least any one of the above temperature-sensitive mutation(s), growth reducing or other attenuating mutation(s), and other mutation(s) as necessary into the viral genome cloned by the methods described above, a known point mutation introduction method such as a homologous recombination method such as double crossover or λ/RED recombination, an overlap PCR method, or a CRISPR/Cas9 method can be used.

Subsequently, the artificial chromosome into which temperature-sensitive mutation(s), growth reducing or other attenuating mutation(s), and other mutation(s) as necessary have been introduced is transfected into host cells to reconstruct recombinant viruses. In the case of the reverse genetics method by the CPER method, fragments into which temperature-sensitive mutation(s), growth reducing or other attenuating mutation(s), and other mutation(s) as necessary have been introduced are assembled by reactions using DNA polymerase, and then transfected into host cells to reconstruct recombinant viruses. The method for transfection is not particularly limited, and a known method can be used. The hosts are also not particularly limited, and known cells can be used.

Subsequently, the reconstructed recombinant virus is infected to cultured cells to subculture the recombinant virus. The cultured cells used at that time are not particularly limited, and examples thereof include Vero cells, VeroE6 cells, Vero cells supplementing expression of TMPRESS2, VeroE6 cells supplementing expression of TMPRESS2, Calu-3 cells, 293T cells supplementing expression of ACE2, BHK cells, 104C1 cells, mouse neuroblastoma-derived NA cells, and Vero cells, and the like. The virus can be recovered by a known method such as centrifugation or membrane filtration. In addition, mass production of recombinant viruses can be made by further adding the recovered viruses to cultured cells.

### EXAMPLES

Hereinafter, the present invention will be described in detail with reference to examples, but the present invention is not limited thereto.

### [Test Example 1: Temperature-sensitive strain of SARS-CoV-2, A50-18 strain (Reference Example)]

### [Test Example 1-1] Isolation of temperature-sensitive strain of SARS-CoV-2, A50-18 strain

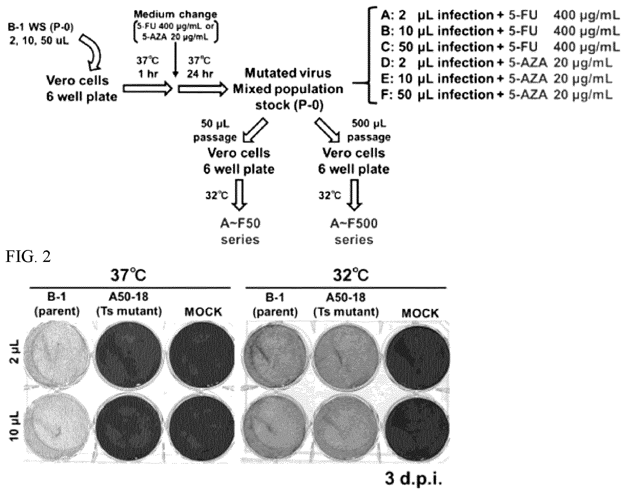

Based on the method of Fig. 1, two types of mutation inducers 5-fluorouracil (hereinafter, 5-FU) and 5-azacytidine (hereinafter, 5-AZA) were added to a clinical isolate of SARS-CoV-2 (B-1 strain [Comparative Example]) (LC603286, also referred to as B-1 strain, a wild-type strain (clinical isolate), European wild-type strain (B-1 strain), B-1 strain (D614G type: pre-alpha European strain) in the present example) to obtain virus populations of A to F50 series and A to F500 series that were adapted at 32°C. Furthermore, passaging of each virus population was carried out a plurality of times, and from among the obtained 406 candidate strains, a virus strain (A50-18 strain. Hereinafter, the strain may be referred to as a Ts strain.) that could grow at 32°C but have remarkably reduced growth at 37°C was found, isolated, and selected (Fig. 2).

### [Test Example 1-2] Analysis of temperature-sensitive strain, A50-18 strain, by next-generation sequencing

### (1-2-1) Mutation analysis of each virus strain

Mutation analysis of the following virus strains was carried out using a next-generation sequencer. The analysis was carried out by extracting RNA from culture supernatants of Vero cells infected with SARS-CoV-2. As a reference, Wuhan-Hu-1 (NC045512), a Wuhan clinical isolate, was used.
B-1: Wild-type strain (clinical isolate)
A50-18: Temperature-sensitive strain
F50-37: Non-temperature-sensitive strain
C500-1: Non-temperature-sensitive strain
F500-53: Non-temperature-sensitive strain
F500-40: Non-temperature-sensitive strain
F500-2: Non-temperature-sensitive strain
(Viruses other than B-1 were mutation-induced viruses)

### (1-2-2) Mutations of temperature-sensitive strain

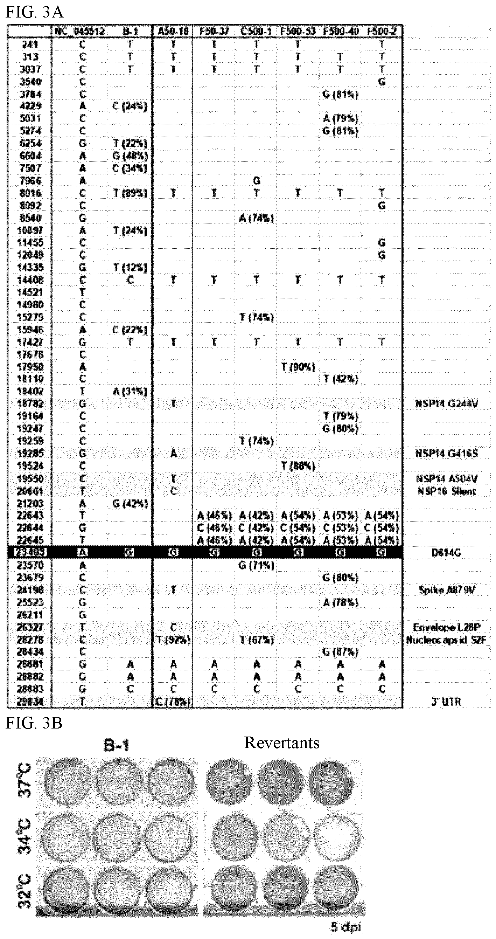

From (1-2-1), the analysis results in Fig.3A were obtained. Since a point mutation of D614G is also observed in B-1 strain, this point mutation is not specific for the temperature-sensitive strain. On the other hand, as characteristic point mutations of the temperature-sensitive strain (A50-18), G248V, G416S, and A504V in NSP14, A879V in Spike, L28P in Envelope, and S2F in Nucleocapsid were identified.

### (1-2-3) Analysis of revertant 1

The "reverse mutation" refers to changing back to the same phenotype as that of the parent virus, which is yet to be mutated, by occurring further mutations into mutated viruses. As used herein, the "reverse mutation" means that an additional mutation occurs into a temperature-sensitive strain, so that a temperature-sensitive characteristic is lost. The additional mutation includes a situation that an amino acid at a site of mutation which is responsible for temperature sensitivity changes back to the amino acid which is yet to be mutated.

As a result of infecting Vero cells with B-1 strain or A50-18 strain at MOI = 0.01 and evaluating growth at 32°C, 34°C and 37°C, samples in which the growth at 37°C was recovered (hereinafter referred to as "revertants") were found out from A50-18 strain. In the revertants, it is considered that among the mutations possessed by the temperature-sensitive strain (A50-18 strain) that had acquired temperature sensitivity, some amino acid residues changed back to the amino acids which is yet to be mutated (hereinafter simply referred to as "reverse mutation"), so that the temperature sensitivity decreased, and the growth at 37°C was recovered. CPE images showing this are shown in Fig. 3B. When the sequences of the obtained samples were confirmed, it was revealed that G248V mutation in NSP14 reverted to mutation to the wild-type G, whereas G416S and A504V mutations in NSP14 and L28P mutation in Envelope were maintained. This suggested that the G248V mutation in NSP14 was a responsible mutation that contributed to temperature sensitivity (temperature-sensitive mutation).

### (1-2-4) Analysis of reverse mutation in isolate 2

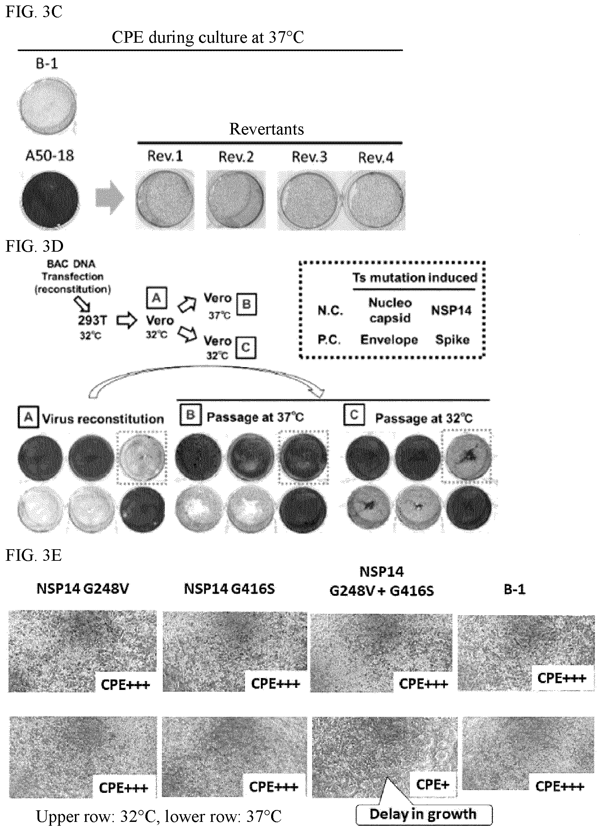

Vero cells were infected with A50-18 strain at moi = 1, and growth at 37°C and 38°C was evaluated. As a result, samples in which the growth at 37°C and 38°C was recovered (revertants) were found. CPE images after culturing the obtained revertants at 37°C for 3 days are shown in Fig. 3C. When the sequences of the revertants were confirmed, the following two types of reversion patterns <1> and <2> were found.

### <1> G248V mutation in NSP14 reverted to mutation to the wild-type G, whereas G416S and A504V mutations were maintained.

### <2> G416S mutation in NSP14 reverted to mutation to the wild-type G, whereas G248V and A504V mutations were maintained.

Since temperature sensitivity was lost when at least one of G248V and G416S in NSP14 reverted to mutation, it was suggested that a combination of the G248V mutation and the G416S mutation in NSP14 was a responsible mutation that contributed to temperature sensitivity (temperature-sensitive mutation).

### (1-2-5) Analysis of recombinant viruses whose mutations were introduced into wild type strain

NSP14, Spike, Nucleocapsid or Envelope derived from A50-18 strain was introduced into BAC DNA having the whole genome of wild-type SARS-CoV-2 by homologous recombination. The obtained recombinant BAC DNA was transfected into 293T cells to reconstruct viruses. Temperature sensitivity was evaluated by infecting Vero cells with the recombinant viruses and observing CPEs at 37°C and 32°C. The results are shown in Fig. 3D. By introducing the NSP14 derived from A50-18 strain, a temperature-sensitive strain showing no CPEs during culture at 37°C was obtained, and thus it was revealed that the NSP14 was a responsible mutation that contributed to temperature sensitivity (temperature-sensitive mutation). On the other hand, since the introduction of Envelope derived from A50-18 strain did not cause temperature sensitivity, it is considered that the mutation in Envelope does not contribute to temperature sensitivity.

### (1-2-6) Analysis of recombinant viruses whose mutations were introduced into wild-type strain 2

Viruses into which the mutations in NSP14 were introduced were reconstructed by the CPER method. Three types of recombinant viruses having the following respective mutations in NSP14 were reconstructed.
· G248V only
· G416S only
· G248V and G416S (hereinafter, also referred to as a "double mutant strain")

Vero cells were infected with each recombinant virus, and CPEs after culture at 37°C or 32°C for 3 days was observed. From Fig. 3-E, it was found that in the viruses only having G248V mutation and the viruses only having G416S mutation, CPEs were observed at 37°C and 32°C similarly to B-1 strain, and therefore these viruses were not temperature-sensitive. On the other hand, in the viruse of the double mutant strain having G248V and G416S mutations, CPE was observed at 32°C, but CPE was only slightly observed at 37°C, that was clearly weaker than CPE at 32°C. From the above results, it was revealed that virus growth was reduced at 37°C by having G248V and G416S mutations, so that the virus was made temperature-sensitive. From this, it was found that a combination of the G248V mutation and the G416S mutation in NSP14 was a responsible mutation that contributed to temperature sensitivity (temperature-sensitive mutation).

### (1-2-7) Analysis of temperature-sensitive strain by Sanger sequencing

Mutation analysis of A50-18 strain was carried out using Sanger sequencing. The analysis was carried out by extracting RNA from culture supernatants of Vero cells infected with SARS-CoV-2. As a result, such a deletion as observed in (2-2-5) of Test Example 2-2 described later was not found.

### (1-2-8) Summary of mutations of temperature-sensitive strains

In the temperature-sensitive strain, A50-18 strain, as shown in Table 2 below, mutations with check marks in the amino acid sequences of the indicated SEQ ID NOs were found, and among them, mutations with double check marks were found as temperature-sensitive mutations. Also, as shown in Table 2 below, it was found that a double mutant strain which only had temperature-sensitive mutations in NSP14 was also a temperature-sensitive strain.

**[Table 2]**

| | Mutation sign | Polypeptide | Corresponding SEQ ID NO** | Amino acid before mutation | Corresponding mutation position*** | Amino acid after mutation | A50-18 (Ts) | Double mutant strain |
|---|---|---|---|---|---|---|---|---|
| | (a) | NSP3 | 1 | V | 404 | A | | |
| [1] | (b) | NSP3 | 1 | L | 445 | F | | |
| | (c) | NSP3 | 1 | K | 1792 | R | | |
| | (d) | NSP3 | 1 | D | 1832 | N | | |
| [1] | (e) | NSP14 | 2 | G | 248 | V | ✔ ✔ | ✔ ✔ |
| | (f) | NSP14 | 2 | G | 416 | S | ✔ ✔ | ✔ ✔ |
| | (g) | NSP14 | 2 | A | 504 | V | ✔ | |
| [1] | (h) | NSP16 | 3 | V | 67 | I | | |
| | (i) | Spike | 4 | L | 54 | W | | |
| | (j) | Spike | 4 | T | 739 | K | | |
| | (k) | Spike | 4 | A | 879 | V | ✔ | |
| | (l) | Envelope | 5 | L | 28 | P | ✔ | |
| | (m) | Nucleocapsid | 6 | S | 2 | F | ✔ | |

| | | | | | | | | |
|---|---|---|---|---|---|---|---|---|
| [1] is temperature-sensitive mutation. ** It shows a sequence in NC 045512 (NCBI) strain, corresponding to a sequence possessed by the actually used strain. *** The mutation position in the sequence possessed by the actually used strain is shown by a position in the sequence of NC 045512 (NCBI) strain. Corresponding sequence and mutation position were identified by sequence alignment. | | | | | | | | |

### [Test Example 1-3] Growth analysis of temperature-sensitive strain, A50-18 strain (1-3-1) Analysis at 32°C and 37°C

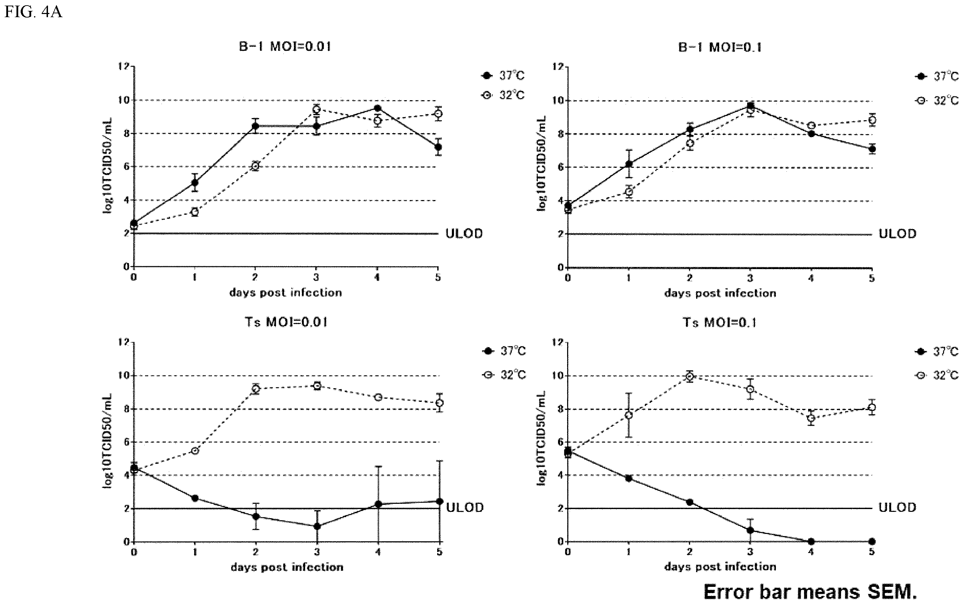

Vero cells were infected with the clinical isolate (B-1 strain [Comparative Example]) and the temperature-sensitive strain (A50-18 strain) using 6-well plates under conditions of MOI = 0.01 or 0.1 (N = 3). After culturing at 37°C or 32°C, each culture supernatant was collected on 0 to 5 dpi. Virus titers of culture supernatants on 0 to 5 dpi were measured by TCID50/mL using the Vero cells. The results are shown in Fig. 4A.

From Fig. 4A, it was found that the virus titer at 3 days after infection at 37°C was not higher than the limit of detection in A50-18 strain, and that growth at 37°C was remarkably reduced.

### (1-3-2) Analysis at 32°C, 34°C and 37°C

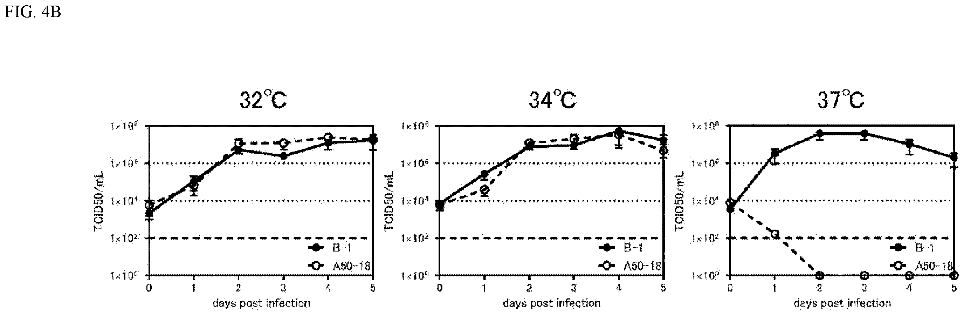

Vero cells were infected with the clinical isolate (B-1 strain [Comparative Example]) and the temperature-sensitive strain (A50-18 strain) using 6-well plates under conditions of MOI = 0.01 (N = 3). After culturing at 37°C, 34°C, or 32°C, each culture supernatant was collected on 0 to 5 dpi. Virus titers of culture supernatants on 0 to 5 dpi were measured by TCID50/mL using the Vero cells. The results are shown in Fig. 4B.

From Fig. 4B, it was found that A50-18 strain grew comparably with the clinical isolate at 32°C and 34°C, whereas growth at 37°C was significantly deficient.

### [Test Example 1-4] Pathogenicity analysis of temperature-sensitive strain, A50-18 strain

### (1-4-1) Weight changes of SARS-CoV-2-infected hamsters

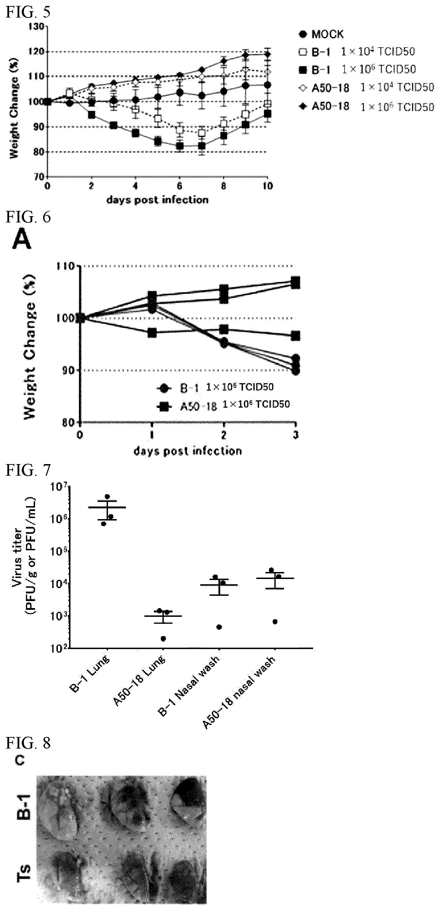

After 4-week-old male Syrian hamsters (n = 4) were bred for 1 week, the clinical isolate (B-1 strain [Comparative Example]) and the temperature-sensitive strain (A50-18 strain) (1 × 10⁴ or 1 × 10⁶ TCID50) were nasally administered at a dose of 100 µL, and weight changes for 10 days were observed. A group to which the same dose of D-MEM medium was nasally administered was defined as a non-infected control (MOCK). The results are shown in Fig. 5.

From Fig. 5, weight loss was not observed when A50-18 strain was infected, so that it was suggested that pathogenicity was significantly low.

### (1-4-2) Viral amounts in lungs or nasal cavities of SARS-CoV-2-infected hamsters

After 4-week-old male Syrian hamsters (n = 3) were bred for 1 week, the clinical isolate (B-1 strain [Comparative Example]) and the temperature-sensitive strain (A50-18 strain) (1 × 10⁶ TCID50) were nasally administered at a dose of 100 µL. The results of observing the weight changes for 3 days are shown in Fig. 6. The hamsters were euthanized on 3 dpi, and then nasal wash was collected with 1 mL of D-PBS. In addition, the lungs of the hamsters were extracted, the right lungs were disrupted, and suspended with 1 mL of D-MEM, and then the supernatants were recovered as lung homogenates by centrifugation. The results of evaluating the viral amounts in these nasal wash and lung homogenates by plaque formation assay using Vero cells are shown in Fig. 7. Furthermore, the extracted left lungs were fixed with 10% formalin and photographed, and the photograph is shown in Fig. 8.

From Fig. 7, it was found that there was no difference in the viral amounts between B-1 strain and A50-18 strain in the nasal wash, whereas the virus amount of A50-18 strain was remarkably low in the lungs. In addition, from Fig. 8, it was found that the hamsters which were infected with B-1 strain and lost the weights showed swelling or blackening in the lungs, whereas A50-18 strain-infected hamsters showed no weight changes and no remarkable changes in the lungs. From the above results, it was estimated that the temperature-sensitive strain was an attenuated strain that could grow in the upper respiratory tract but could not grow in the lower respiratory tract.

### (1-4-3) Histological analysis of SARS-CoV-2-infected hamsters

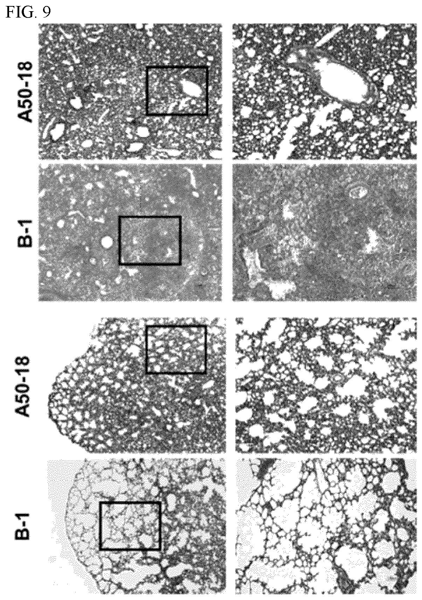

Sections were prepared from the formalin-fixed lungs obtained by the infection experiment to the hamsters carried out in (1-4-2), and HE staining was carried out to analyze histological pathogenicity of the lungs due to SARS-CoV-2 infection. The results are shown in Fig. 9.

As shown in Fig. 9, infiltration of erythrocytes and destruction of alveolar structures were observed in the lungs obtained from the hamsters infected with the clinical isolate (B-1 strain [Comparative Example]). On the other hand, in the lungs of the hamsters infected with the temperature-sensitive strain (A50-18 strain [Reference Example]), such severe pathologies were not observed. This also strongly suggested that A50-18 strain did not induce severe inflammation in the lungs at the time of infection and had low pathogenicity.

### (1-4-4) Histological analysis of SARS-CoV-2-infected hamsters by immunochemical staining

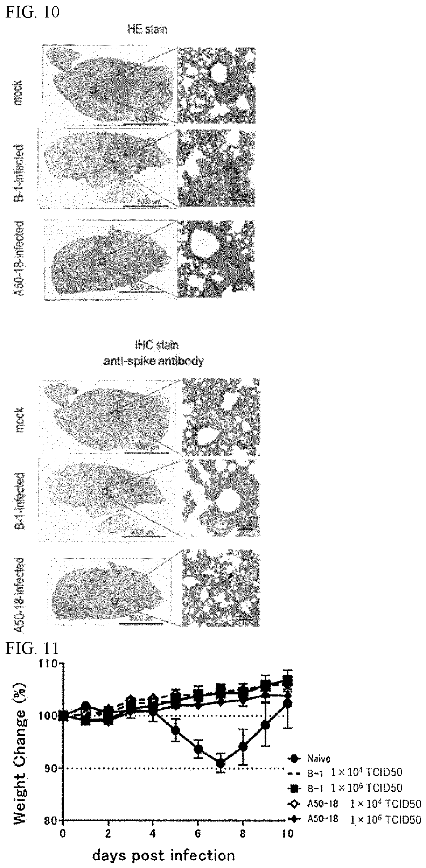

In order to evaluate relationships between virus growth and pathogenicity as to the histological pathogenicity observed in (1-4-3), viral proteins were detected by immunochemical staining. After 4-week-old male Syrian hamsters (B-1, A50-18: n = 5, MOCK: n = 3) were bred for 1 week, the clinical isolate (B-1 strain [Comparative Example]) and the temperature-sensitive strain (A50-18 strain) (1 × 10⁶ TCID50) were nasally administered at a dose of 100 µL. The hamsters were euthanized on 3 dpi, and then the extracted left lungs were fixed with 10% formalin to prepare serial sections. HE staining and immunochemical staining (also referred to as immunohistochemistry (IHC) staining) were carried out using the obtained serial sections. For immunochemical staining, rabbit anti-spike polyclonal antibodies (Sino Biological: 40589-T62) were used. HE staining images and immunochemical staining images are shown in Fig. 10. As in (1-4-3), in B-1 strain-infected hamsters, infiltration of erythrocytes and destruction of alveolar structures were observed, and spike proteins were widely detected by immunochemical staining. On the other hand, in A50-18 strain-infected hamsters, such tissue damages were not observed, and spike proteins were also locally detected only in limited areas. From these results, it was revealed that B-1 strain remarkably grew in the lung tissue and showed tissue damages, whereas A50-18 strain could not efficiently grow in the lung tissue, and lung tissue damages by the strain were low.

### [Test Example 1-5] Immunogenicity analysis of temperature-sensitive strain, A50-18 strain

### (1-5-1) Wild-type strain challenge on temperature-sensitive strain-infected hamsters

According to the following procedure, temperature-sensitive strain-infected hamsters were challenged with a wild-type strain (clinical isolate).

After 4-week-old male Syrian hamsters (n = 4) were bred for 1 week, the clinical isolate (B-1 strain, [Comparative Example]) or the temperature-sensitive strain (A50-18 strain) (1 × 10⁴ or 1 × 10⁶ TCID50) was nasally administered at a dose of 100 µL. After 21 days, the clinical isolate (B-1 strain) (1 × 10⁶ TCID50) was nasally administered again at a dose of 100 µL, and weight changes for 10 days were observed. At this time, non-infected hamsters of the same age (n = 3) were used as a naive control. The results were shown in Fig. 11.

As shown in Fig. 11, the naive hamsters were observed to lose weight due to infection with B-1 strain, whereas the hamsters infected once with B-1 strain or A50-18 strain did not lose weight. This revealed that immunity contributing to protection against infection was induced not only in infection with the wild-type strain, B-1 strain, but also in infection with A50-18 strain having low pathogenicity.

### (1-5-2) Induction analysis of neutralizing antibodies of temperature-sensitive strain-infected hamsters

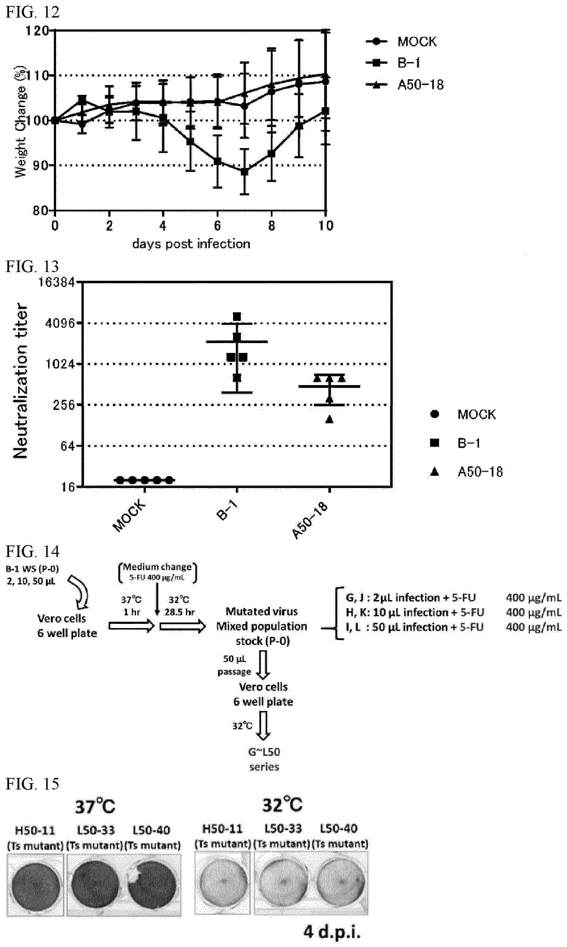

After 4-week-old male Syrian hamsters (n = 5) were bred for 1 week, the clinical isolate (B-1 strain [Comparative Example]) or the temperature-sensitive strain (A50-18 strain) (1 × 10⁶ TCID50) was nasally administered at a dose of 100 µL. Weight changes after infection are shown in Fig. 12. Similar to the previous results, weight loss was observed by infection with B-1 strain, whereas no weight loss was observed by infection with A50-18 strain.

21 days after infection, whole blood was collected, and serum was separated, and then the serum was inactivated by heating at 56°C for 30 minutes. B-1 strain of 100 TCID50 was mixed with inactivated serum which had been serially diluted, and the mixture was reacted at 37°C for 1 hour. After the reaction, the culture mixture was seeded on Vero cells, and after culturing at 37°C, neutralizing activity of the virus was evaluated by observing CPE. The highest dilution rate which did not cause CPE was defined as a neutralizing antibody titer. The results are shown in Fig. 13. Serum of the non-infected hamsters did not show neutralizing activity against B-1 strain, whereas serum of the hamsters infected withB-1 strain or A50-18 strain could induce neutralizing antibodies.

### [Test Example 2: SARS-CoV-2 temperature-sensitive strains, H50-11 strain, L50-33 strain, and L50-40 strain (Examples)]

### [Test Example 2-1] Additional isolation of SARS-CoV-2 temperature-sensitive strains, H50-11 strain, L50-33 strain, and L50-40 strain

For the purpose of isolation of further candidate strains, temperature-sensitive strains were isolated by the method of Fig. 14. Vero cells were infected with clinical isolate of SARS-CoV-2 (B-1 strain [Comparative Example]), and in a state where a mutation inducer 5-FU was added, virus populations of G to L50 series that were adapted at 32°C were obtained. Furthermore, passaging of each virus population was carried out a plurality of times, and from among the obtained 253 strains, virus strains that can could grow at 32°C but remarkably reduced the growth at 37°C (H50-11 strain, L50-33 strain, L50-40 strain) were found, isolated, and selected (Fig. 15).

[Test Example 2-2] Analysis of additional temperature-sensitive isolates, H50-11 strain, L50-33 strain, and L50-40 strain, by next-generation sequencing

### (2-2-1) Mutation analysis method for additional isolates

Mutation analysis of the following virus strains was carried out using a next-generation sequencer. The analysis was carried out by extracting RNA from culture supernatants of Vero cells infected with SARS-CoV-2. As a reference, Wuhan-Hu-1 (NC045512), a Wuhan clinical isolate, was used.
H50-11 strain: Temperature-sensitive strain
L50-33 strain: Temperature-sensitive strain
L50-40 strain: Temperature-sensitive strain

### (2-2-2) Results of mutation analysis of temperature-sensitive strains

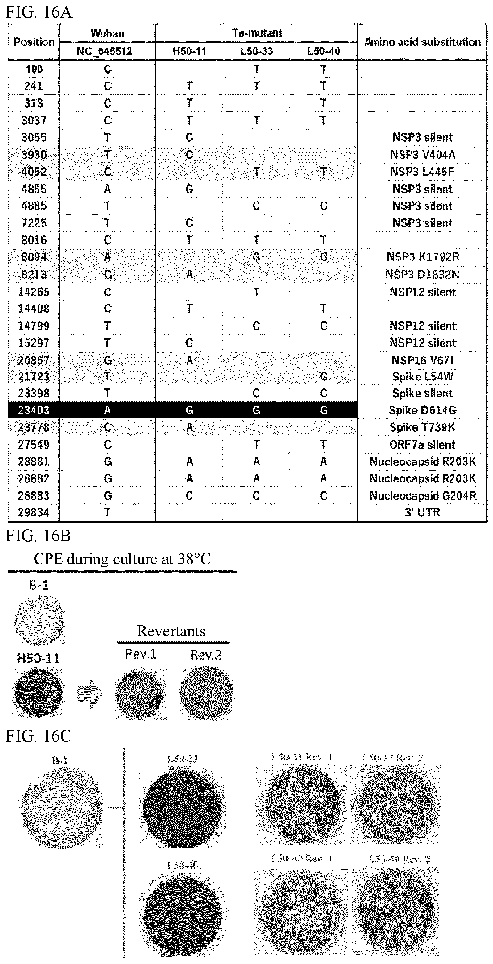

From (2-2-1), the analysis results in Fig. 16A were obtained. As characteristic point mutations of H50-11 strain, V404A and D1832N in NSP3, V67I in NSP16, and T739K in Spike were found. In addition, as characteristic point mutations of L50-33 strain, L445F and K1792R in NSP3 were found, and as characteristic point mutations of L50-40 strain, L445F and K1792R in NSP3 and L54W in Spike were found.

### (2-2-3) Analysis of revertants of additional isolate (H50-11 strain)

Vero cells were infected with H50-11 strain at moi = 1, and growth at 37°C and 38°C were evaluated. As a result, samples in which the growth at 37°C and 38°C was recovered (revertants) were found. CPE images after culturing the obtained revertants at 38°C for 3 days are shown in Fig. 16B. When the sequences of the obtained samples were confirmed, V67I mutation in NSP16 reverted to mutation to the wild-type V, whereas the other amino acid mutations were maintained. This suggested that V67I mutation in NSP16 was a responsible mutation that contributed to temperature sensitivity (temperature-sensitive mutation).

### (2-2-4) Analysis of revertants of additional isolates (L50-33 strain and L50-40 strain)

Vero cells were infected with L50-33 strain and L50-40 strain at MOI = 0.01, and growth at 32°C, 34°C and 37°C was evaluated. As a result, from among L50-33 strain and L50-40 strain, samples in which the growth at 37°C was recovered (hereinafter, revertants) were found. CPE images after culturing each strain at 37°C for 3 days are shown in Fig. 16C. The revertants of L50-33 strain and L50-40 strain are indicated as L50-33 strain Rev1 and 2 and L50-40 strain Rev1 and 2, respectively. When the sequences of the obtained samples were confirmed, it was revealed that L445F mutation in NSP3 was mutated to wild-type L or C, whereas K1792R mutation in NSP3 was maintained. This suggested that L445F mutation in NSP3 might be a responsible mutation that contributed to temperature sensitivity (temperature-sensitive mutation).

### (2-2-5) Analysis of temperature-sensitive strains by Sanger sequencing

Mutation analysis of H50-11 strain, L50-33 strain, and L50-40 strain was carried out using Sanger sequencing. The analysis was carried out by extracting RNA from culture supernatants of Vero cells infected with SARS-CoV-2.

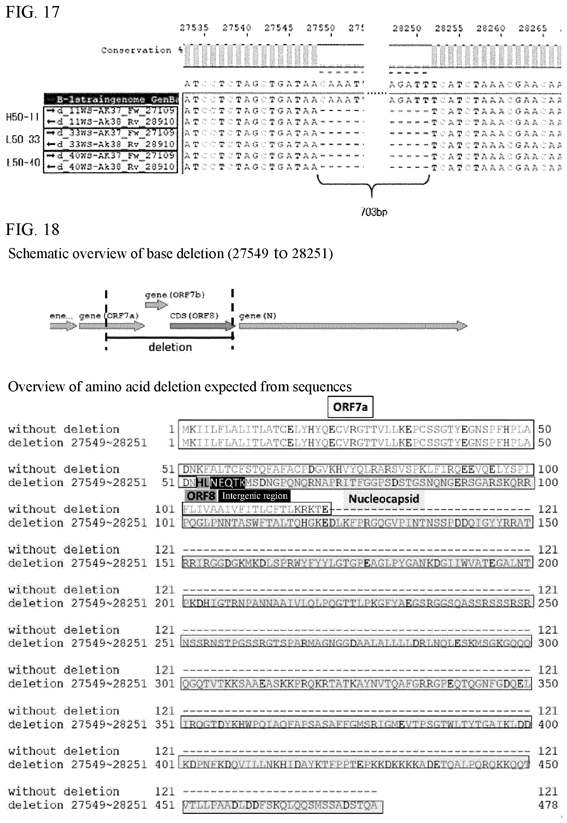

As a result, a deletion of a base sequence (SEQ ID NO: 7) at positions 27549 to 28251 as shown in Fig. 17 was found in all three strains. A schematic overview of the deletion of the base sequence at positions 27549 to 28251 and a deletion of an amino acid sequence encoded thereby is shown in Fig. 18. Also, in Fig. 18, ORF7a is a base sequence at positions 27394 to 27759, ORF7b is a base sequence at positions 27756 to 27887, and ORF8 is a base sequence at positions 27894 to 28259.

As shown in Fig. 18, the base sequence region at positions 27549 to 28251 corresponds to a portion of ORF7a (an amino acid sequence from positions 53 to the terminal end. The same applies hereinafter.), the entire ORF7b, and most of the amino acid sequence of ORF8. Since the deletion of this region involves a frameshift, it was considered that a protein was produced in which an amino acid sequence at positions 1 to 52 of ORF7a was fused with an amino acid sequence encoded by eight bases at the 3' terminal of ORF8, an intergenic region and a base sequence of Nucleocapsid. In addition, ORF7b was entirely deleted, and the original sequence of ORF8 was also entirely deleted.

### (2-2-6) Summary of mutations of temperature-sensitive strains

In the temperature-sensitive strains, H50-11 strain, L50-33 strain, and L50-40 strain, as shown in Table 3 below, mutations with check marks in the amino acid sequences of the indicated SEQ ID NOs were found, and among them, mutations with double check marks were found as temperature-sensitive mutations.

**[Table 3]**

| | Mutation sign | Polypeptide | Corresponding SEQ ID NO** | Amino acid before mutation | Corresponding mutation position*** | Amino acid after mutation | H50-11 | L50-33 | L50-40 |
|---|---|---|---|---|---|---|---|---|---|
| | (a) | NSP3 | 1 | V | 404 | A | ✔ | | |
| [1] | (b) | NSP3 | 1 | L | 445 | F | | ✔✔ | ✔✔ |
| | (c) | NSP3 | 1 | K | 1792 | R | | ✔ | ✔ |
| | (d) | NSP3 | 1 | D | 1832 | N | ✔ | | |
| [1] | (e) | NSP14 | 2 | G | 248 | V | | | |
| | (f) | NSP14 | 2 | G | 416 | S | | | |
| | (g) | NSP14 | 2 | A | 504 | V | | | |
| [1] | (h) | NSP16 | 3 | V | 67 | I | ✔✔ | | |
| | (i) | Spike | 4 | L | 54 | W | | | ✔ |
| | (j) | Spike | 4 | T | 739 | K | ✔ | | |
| | (k) | Spike | 4 | A | 879 | V | | | |
| | (l) | Envelope | 5 | L | 28 | P | | | |
| | (m) | Nucleocapsid | 6 | S | 2 | F | | | |
| [2] | (n) | ORF7a-8 | 7 | (Complete) | 1-703 | (Deletion) | ✔ | ✔ | ✔ |

| | | | | | | | | | |
|---|---|---|---|---|---|---|---|---|---|
| [1] is temperature-sensitive mutation, and [2] is growth reducing or other attenuating mutation. ** It shows a sequence in NC 045512 (NCBI) strain, corresponding to a sequence possessed by the actually used strain *** The mutation position in the sequence possessed by the actually used strain is shown by a position in the sequence of NC 045512 (NCBI) strain. Corresponding sequence and mutation position were identified by sequence alignment. | | | | | | | | | |

### [Test Example 3] Growth analysis of additional isolates, H50-11 strain, L50-33 strain, and L50-40 strains (Examples)

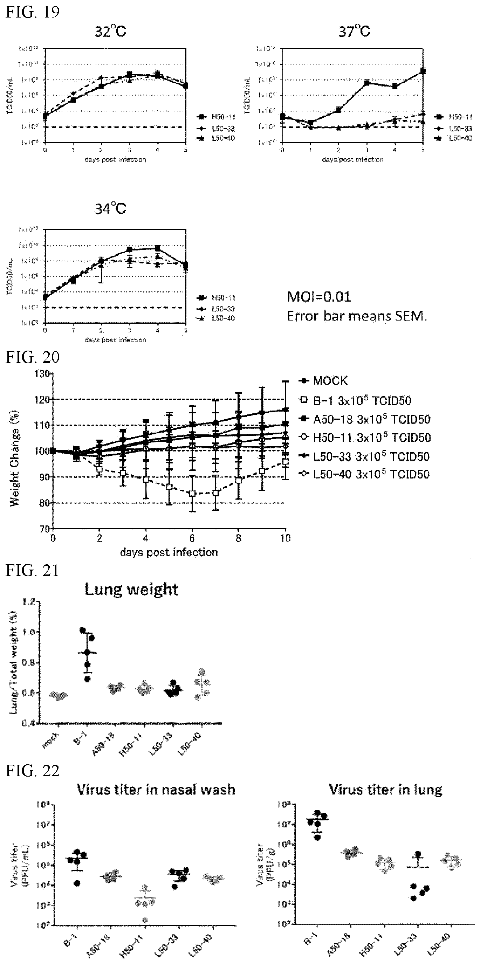

Vero cells were infected with additional isolates under the condition of MOI = 0.01 (N = 3). After culturing at 37°C, 34°C or 32°C, each culture supernatant was collected on 0 to 5 d.p.i. Virus titers of these culture supernatants were measured by TCID₅₀/mL using the Vero cells. The results are shown in Fig. 19. As a result, the obtained additional isolates showed growth at 32°C and 34°C, whereas their growth was reduced at 37°C.

### [Test Example 4] Pathogenicity analysis of each temperature-sensitive strain (4-1) Weight changes of temperature-sensitive strain-infected hamsters

After 4-week-old male Syrian hamsters (n = 5) were bred for 1 week, the clinical isolate (B-1 strain [Comparative Example]) or the temperature-sensitive strains (A50-18 strain [Reference Example], and L50-33 strain, L50-40 strain, and H50-11 strain [Examples]) (3 × 10⁵ TCID50) were nasally administered at a dose of 100 µL, and weight changes for 10 days were observed. A group to which the same dose of D-MEM medium was nasally administered was defined as a non-infected control (MOCK). The results are shown in Fig. 20. The hamsters infected with B-1 strain lost weight about 20% in 7 days, whereas the hamsters infected with the temperature-sensitive strains did not lose weight significantly in all groups, so that it was suggested that pathogenicity was significantly low.

### (4-2) Viral amounts in lungs or nasal cavities of temperature-sensitive strain-infected hamsters

After 4-week-old male Syrian hamsters (n = 5) were bred for 1 week, the clinical isolate (B-1 strain [Comparative Example]) or the temperature-sensitive strains (A50-18 strain [Reference Example], and L50-33 strain, L50-40 strain, and H50-11 strain [Examples]) (3 × 10⁵ TCID50) were nasally administered at a dose of 100 µL. The hamsters were euthanized on 3 dpi, and then nasal wash was collected with 1 mL of D-PBS. In addition, the lungs of the hamsters were extracted, and the lung weight was measured. Thereafter, the right lungs were disrupted, and suspended with 1 mL of D-MEM, and then the supernatants were recovered as lung homogenates by centrifugation. The lung weight per total weight of the hamsters is shown in Fig. 21. In addition, the results of evaluating the viral amounts in these nasal wash and lung homogenates by plaque formation assay using Vero cells are shown in Fig. 22.

As a result of comparing lung weight per total weight of hamsters, the lung weight of B-1 strain-infected hamsters increased, and it was strongly suggested that the lungs swelled due to inflammation or the like. On the other hand, such an increase in lung weight was not observed in the temperature-sensitive strain-infected hamsters. In addition, as a result of comparing viral amounts in the nasal wash, there was no significant difference between B-1 strain-infected hamsters and the hamsters infected with the temperature-sensitive strains except H50-11 strain, but H50-11 strain-infected hamsters had a small viral amount in the nasal wash. Furthermore, it was revealed that the intrapulmonary viral amounts of the temperature-sensitive strain-infected hamsters were significantly lower than those of B-1 strain-infected hamsters. From these results, it was speculated that each temperature-sensitive strain was an attenuated strain that could not grow in the lower respiratory tract, similarly to A50-18 strain in Test Example 1.

### [Test Example 5] Immunogenicity analysis of each temperature-sensitive strain (5-1) Wild-type strain challenge on temperature-sensitive strain-infected hamsters

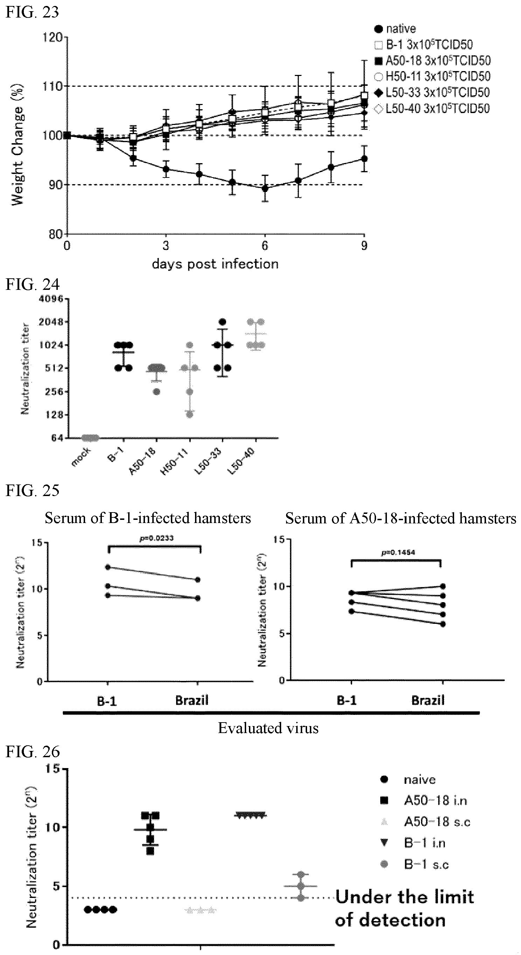

After 4-week-old male Syrian hamsters (n = 5) were bred for 1 week, the clinical isolate (B-1 strain [Comparative Example]) or the temperature-sensitive strains (A50-18 strain [Reference Example], and L50-33 strain, L50-40 strain, and H50-11 strain [Examples]) (3 × 10⁵ TCID50) were nasally administered at a dose of 100 µL. After 21 days, the clinical isolate (B-1 strain) (3 × 10⁵ TCID50) was nasally administered again at a dose of 100 µL, and weight changes for 9 days were observed. At this time, non-infected hamsters of the same age (n = 5) were used as a naive control. The results are shown in Fig. 23. The naive hamsters were observed to lose weight due to infection with B-1 strain, whereas the hamsters infected once with B-1 strain and each temperature-sensitive strain did not lose weight. This revealed that immunity contributing to protection against infection could be induced not only in infection with the wild-type strain, B-1 strain, but also in infection with each temperature-sensitive strain having low pathogenicity.

### (5-2) Induction analysis of neutralizing antibodies of temperature-sensitive strain-infected hamsters

After 4-week-old male Syrian hamsters (n = 5) were bred for 1 week, the clinical isolate (B-1 strain [Comparative Example]) or the temperature-sensitive strains (A50-18 strain [Reference Example], and L50-33 strain, L50-40 strain, and H50-11 strain [Examples]) (3 × 10⁵ TCID50) were nasally administered at a dose of 100 µL. After 20 days, blood was partially collected, and the obtained serum was used to measure neutralizing activity against the clinical isolate (B-1 strain). The neutralizing activity was measured in the same method as in (1-5-2). The measurement results are shown in Fig. 24. It was revealed that antibodies having neutralizing activity were induced not only in the hamsters infected with B-1 strain but also in the hamsters infected with each temperature-sensitive strain.

### [Test Example 6] Efficacy analysis on SARS-CoV-2 mutant strains (Reference Example)

Evaluation of neutralizing activity of serum of temperature-sensitive strain-infected hamsters against SARS-CoV-2 mutant strains

After 4-week-old male Syrian hamsters (n = 3 or 5) were bred for 1 week, the clinical isolate (B-1 strain [Comparative Example]) or the temperature-sensitive strain (A50-18 strain [Reference Example]) (3 × 10⁵ TCID50) was nasally administered at a dose of 100 µL. Blood was partially collected from hamsters at 3 weeks after infection, and the obtained serum was used to measure the neutralizing activity against live viruses of the SARS-CoV-2 European clinical isolate (B-1) and a Brazilian variant (hCoV-19/Japan/TY7-503/2021 strain). The results are shown in Fig. 25. The neutralizing activity was measured in the same method as in (1-5-2). It was revealed that the hamsters infected with B-1 strain or the temperature-sensitive strain also showed neutralizing activity against the Brazilian variant. From this, it was speculated that the present live attenuated vaccine was also effective against the SARS-CoV-2 mutant strains.

### [Test Example 7] Comparative study experiments on administration routes and doses (Reference Example)

### (7-1) Comparison of immunogenicity by administration routes

After 4-week-old male Syrian hamsters (n = 5) were bred for 1 week, the clinical isolate (B-1 strain [Comparative Example]) or the temperature-sensitive strain (A50-18 strain [Reference Example]) (3 × 10⁵ TCID50) was nasally or subcutaneously administered at a dose of 100 µL. The untreated group was defined as a naive control. After 3 weeks, the serum obtained by partial blood collection from the hamsters was used to evaluate neutralizing activity against the SARS-CoV-2 Brazilian variant (hCoV-19/Japan/TY7-503/2021 strain). The neutralizing activity was measured in the same method as in (1-5-2). The results of the neutralizing activity are shown in Fig. 26. "i.n" denotes nasal administration, and "S.C" denotes subcutaneous administration. By nasal administration of B-1 strain and A50-18 strain, neutralizing antibodies against live viruses could be induced. For subcutaneous administration, almost no neutralizing antibodies could be induced at the tested doses, but in view of the results of nasal administration, it was considered that neutralizing antibodies could be induced even by subcutaneous administration when the dose was increased.

### (7-2) Comparison of immunogenicity by doses

After 4-week-old male Syrian hamsters (n = 5) were bred for 1 week, the temperature-sensitive strain (A50-18 strain) was administered nasally or subcutaneously. Doses are shown in Table 4.

**[Table 4]**

| | 1^{st} infection | |
|---|---|---|
| | Inoculation titer | Route |
| mock (n.c.) | | |
| Robust titer infection (p.c.) | 3.0 × 10⁵ TCID₅₀ | i.n. 100 µL |
| High dose i.n. | 1.0 × 10⁴ TCID₅₀ | i.n. 10 µL |
| Low dose i.n. | 1.0 × 10² TCID₅₀ | i.n. 10 µL |
| High dose s.c. | 1.0 × 10⁴ TCID₅₀ | s.c. 100 µL |
| Low dose s.c. | 1.0 × 10² TCID₅₀ | s.c. 100 µL |

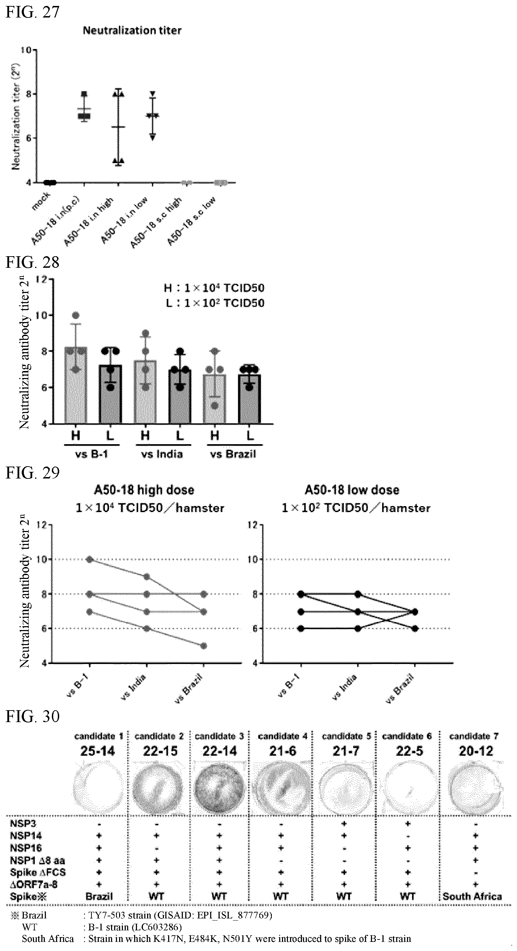

Blood was partially collected from hamsters at 3 weeks after infection, and the obtained serum was used to measure the neutralizing activity against live viruses of the SARS-CoV-2 Brazilian variant (hCoV-19/Japan/TY7-503/2021 strain). The results are shown in Fig. 27. "i.n" denotes nasal administration, and "S.C" denotes subcutaneous administration. The neutralizing activity was measured in the same method as in (1-5-2). Similarly to (7-1), by the nasal administration, an increase in neutralizing antibody titer was observed even in the low dose administration group of 1 × 10² TCID50/10 µL. This suggested that the temperature-sensitive strain could induce sufficient immunity even by nasal administration of a small amount. For subcutaneous administration, almost no neutralizing antibodies could be induced at the tested doses, but in view of the results of nasal administration, it was considered that neutralizing antibody could be induced even by subcutaneous administration when the dose was increased.

### [Test Example 8] Efficacy analysis on SARS-CoV-2 mutant strains (Reference Example)

After 4-week-old male Syrian hamsters (n = 4) were bred for 1 week, the temperature-sensitive strain (A50-18 strain [Reference Example]) of 1 × 10⁴ TCID50 or 1 × 10² TCID50 was nasally administered at a dose of 10 µL. Blood was partially collected from hamsters at 3 weeks after infection, and the obtained serum was used to measure the neutralizing activity against live viruses of the SARS-CoV-2 European wild-type strain (B-1 strain), an Indian variant (self-isolated strain), and the Brazilian variant (hCoV-19/Japan/TY7-503/2021 strain). The results are shown in Fig. 28.

The neutralizing activity was measured by the same method as in (1-5-2). It was revealed that an individual to which not only the parent wild-type B-1 strain, but also A50-18 strain was nasally administered in a small amount could induce neutralizing antibodies against the Indian variant and the Brazilian variant in a dose-dependent manner.

Moreover, the results of comparing neutralizing antibody titers against each strain of the serum of each individual are shown in Fig. 29. It was revealed that all individuals possessed neutralizing antibodies, although some individuals exhibited a decrease in neutralizing antibody titer against the Brazilian variant. From these results, it was suggested that immunity by nasal administration of the temperature-sensitive strains might exhibit cross-protection.

### [Test Example 9] Creation of vaccine candidate strains by CPER reaction (Examples)

Since live attenuated vaccines inherently involve growth in a host's body, there is a possibility that a mutation that occurs during nucleic acid replication results in the development of a virulent wild-type strain. In order to reduce the possibility, strains were constructed in which temperature-sensitive mutations and growth reducing or other attenuating mutations were combined. Such strains were constructed so as to be able to maintain their attenuation even when the temperature-sensitive mutations were lost. As temperature-sensitive mutations, growth reducing or other attenuating mutations, and other mutations, mutations checked in Table 5 [i.e., L445F in NSP3, G248V and G416S in NSP14, and V67I in NSP16; deletion of eight amino acids at positions 32 to 39 in NSP1, deletion of furin cleavage site (FCS) in Spike (specifically, the deletion at the 679th to 686th positions of the spike and V687I), and loss of function in ORF8; any of the other mutations found in the temperature-sensitive strains (NSP3 K1792R and NSP14 A504V)] were used.

**[Table 5]**

| | Mutation sign | Polypeptide | Corresponding SEQ ID NO** | Amino acid before mutation | Corresponding mutation position*** | Amino acid after mutation | Candidate strain 1 | Candidate strain 2 | Candidate strain 3 | Candidate strain 4 | Candidate strain 5 | Candidate strain 6 | Candidate strain 7 |
|---|---|---|---|---|---|---|---|---|---|---|---|---|---|
| | (a) | NSP3 | 1 | V | 404 | A | | | | | | | |
| [1] | (b) | NSP3 | 1 | L | 445 | F | | | | | ✔ | ✔ | |
| | (c) | NSP3 | 1 | K | 1792 | R | | | | | ✔ | ✔ | |
| | (d) | NSP3 | 1 | D | 1832 | N | | | | | | | |
| [1] | (e) | NSP14 | 2 | G | 248 | V | ✔ | ✔ | ✔ | ✔ | ✔ | | ✔ |
| | (f) | NSP14 | 2 | G | 416 | S | ✔ | ✔ | ✔ | ✔ | ✔ | | ✔ |
| | (g) | NSP14 | 2 | A | 504 | ν | ✔ | ✔ | ✔ | ✔ | ✔ | | ✔ |
| [1] | (h) | NSP16 | 3 | V | 67 | I | ✔ | | ✔ | ✔ | | ✔ | ✔ |
| | (i) | Spike | 4 | L | 54 | W | | | | | | | |
| | (j) | Spike | 4 | T | 739 | K | | | | | | | |
| | (k) | Spike | 4 | A | 879 | V | | | | | | | |
| | (l) | Envelope | 5 | L | 28 | P | | | | | | | |
| | (m) | Nucleocapsid | 6 | S | 2 | F | | | | | | | |
| [2] | (n) | ORF7a-8 | 7 | (Complete) | 1-703 | (Deletion) | ✔ | ✔ | ✔ | ✔ | ✔ | ✔ | ✔ |
| [2] | (o) | Spike | 4 | (Complete) | 681-684 | (Deletion) | | ✔ | ✔ | ✔ | ✔ | ✔ | |
| | (o) | Spike | 4 | (Complete) | 681-684 | (Deletion) | ✔ | | | | | | |
| | (P) | Spike | 4 | (Complete) | 679-680 | (Deletion) | | ✔ | ✔ | ✔ | ✔ | ✔ | |
| | | | | | 685-686 | (Deletion) | | ✔ | ✔ | ✔ | ✔ | ✔ | |
| | (P) | Spike | 4 | (Complete) | 679-680 | (Deletion) | ✔ | | | | | | |
| | | | | | 685-686 | (Deletion) | ✔ | | | | | | |
| | (q) | Spike | 4 | V | 687 | I | | ✔ | ✔ | ✔ | ✔ | ✔ | |
| | (q) | Spike | 4 | V | 687 | I | ✔ | | | | | | |
| [2] | (r) | NSP1 | 8 | (Complete) | 32-39 | (Deletion) | ✔ | ✔ | ✔ | | | | ✔ |
| | Original strain of spike sequence* | | | | | | Br | WT | WT | WT | WT | WT | SA |
| | Original strain of sequence other than spike sequence* | | | | | | WT | WT | WT | WT | WT | WT | WT |

| | | | | | | | | | | | | | |
|---|---|---|---|---|---|---|---|---|---|---|---|---|---|
| [1] is temperature-sensitive mutation, and [2] is growth reducing or other attenuating mutation. *Details of the original strains of sequences are as shown below: WT = Wild type (B-1 strain; LC603286) Br = Brazilian type (GISAID: EPI_ISL_877769) SA = South African strain (strain in which K417N, E484K, N501Y were introduced to B-1 strain) ** Regarding the sequence other than the spike sequence, it shows a sequence in NC_045512 (NCBI) strain, corresponding to a sequence possessed by WT. Regarding the spike sequence, it shows a sequence in NC_045512 (NCBI) strain, corresponding to a sequence possessed by WT, Br or SA. *** The mutation position in the WT, Br or SA sequence actually used is shown by a position in the sequence of NC_045512 (NCBI) strain. Corresponding sequence and mutation position were identified by sequence alignment. | | | | | | | | | | | | | |

By a reverse genetics method using CPER, strains having the above temperature-sensitive mutations and growth reducing or other attenuating mutations together with other mutations were constructed (Torii et al. cell report 2020). The genome of SARS-CoV-2 B-1 strain was fragmented and cloned into a plasmid. Inverse PCR was used to introduce the mutations of interest into the cloned fragments. Using the plasmid obtained by cloning the wild-type fragment as a template, a SARS-CoV-2 wild-type genomic fragment was obtained by PCR. In addition, PCR or RT-PCR was carried out using the plasmid into which the mutations were introduced or the genome of the SARS-CoV-2 mutant strains having the mutations of interest as a template, so as to obtain SARS-CoV-2 mutant genomic fragments.

The obtained fragments and linker fragments containing a CMV promoter were mixed, and CPER was carried out using PrimeStar GXL polymerase to circularize a plurality of fragments. The reaction mixture was transfected into BHK/hACE2 cells, and the cells were cultured at 34°C to reconstruct target viruses. The culture supernatant of the obtained cells was added to VeroE6/TMPRSS II cells and cultured at 34°C to recover the reconstructed viruses. The temperature-sensitive strain mutation(s) and/or growth reducing or other attenuating mutation(s) introduced into each candidate strain and CPE images are shown in Fig. 30. CPEs were observed in the VeroE6/TMPRSS II cells, revealing that the viruses were reconstructed.

### [Test Example 10] Evaluation of temperature sensitivity of candidate strains (Examples)

### (10-1) Temperature sensitivity of candidate strains 1 to 7

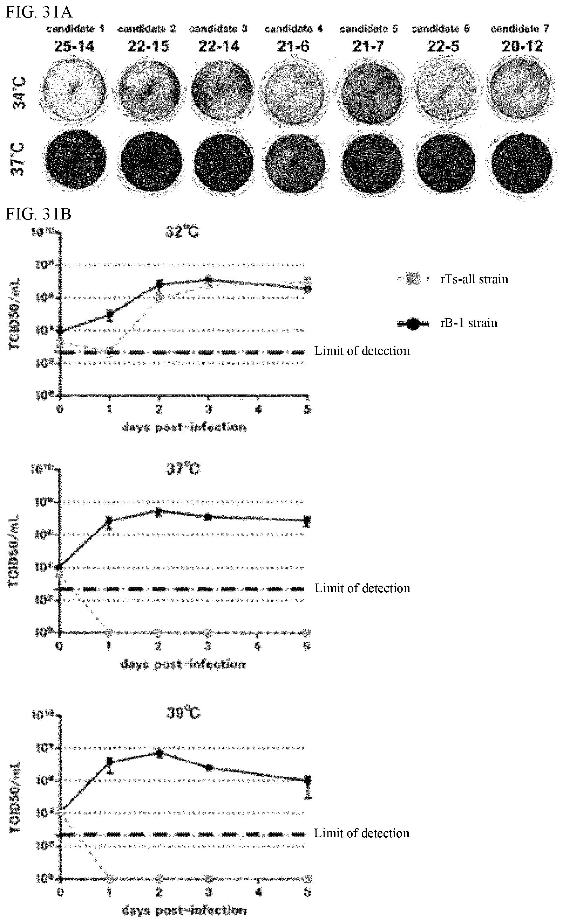

In order to evaluate the temperature sensitivity of candidate strains 1 to 7 (Examples) obtained in Test Example 9, Vero cells were infected with 2 µL of supernatant of recovery culture of each candidate strain, and growth at 34°C and 37°C was compared. CPE images after culturing for 3 days are shown in Fig. 31A. All strains showed CPE at 34°C, whereas none of the candidate strains 1, 2, 3, 6 and 7 showed CPE at 37°C. Candidate strains 4 and 5 showed slightly CPE, but more weakly than that at 34°C. This confirmed that the vaccine candidate strains exhibited temperature sensitivity.

### (10-2) Temperature sensitivity of strain reconstructed by introducing all temperature-sensitive mutations

A strain reconstructed by introducing all temperature-sensitive mutations (rTs-all strain [Example]) was obtained. In rTs-all strain, three types: a mutation of (b), a combination of mutations of (e) and (f), and a mutation of (h), are introduced as temperature-sensitive mutations, and only a mutation of (n) is introduced as other attenuating mutation, and none of other mutations is introduced.

Vero cells were infected with rTs-all strain at MOI = 0.01, cultured at 32, 37 or 39°C for 5 days, and culture supernatants were sampled over time (n = 3). Virus titers at each time point were measured by TCID50 method. For comparison, this B-1 strain (rB-1 strain) prepared by the CPER method was used. The resulting growth curves are shown in Fig. 31B. As shown in Fig. 31B, rTs-all strain grew at 32°C, whereas the growth at 37°C or 39°C was remarkably reduced (below the limit of detection at day 1, and the strain did not grow thereafter). Since the growth at 32°C of rTs-all strain was slightly delayed as compared with that of the rB-1 strain, it is recognized that rTs-all strain more strongly expressed than the preferred degree (i.e., the growth at 32°C is comparable with that of the rB-1 strain) as the level of temperature sensitivity. From this result, it was found that, in order to control the level of temperature sensitivity to the preferred degree, it was preferable to select one or two types rather than three types, among the temperature-sensitive mutations (that is, three types of mutations or combinations of mutations: the mutation of (b), the combination of mutations of (e) and (f), and the mutation of (h)).

### [Test Example 11] Evaluation of immunogenicity at low titer and low dose of candidate strains 1, 3, 4, 6, and 7 (neutralizing antibody titer induction) (Examples)

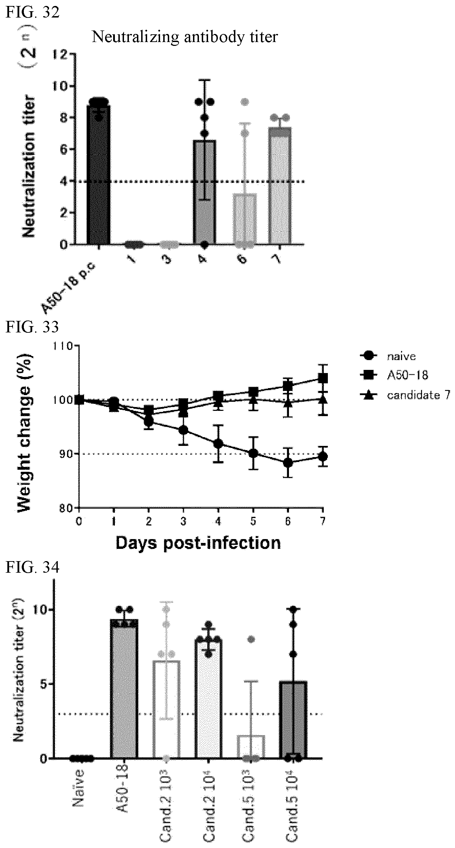

In order to evaluate the immunogenicity of the candidate strains 1, 3, 4, 6 and 7 (Examples) among the candidate strains obtained in Test Example 9, 10 µL of each candidate strain of 100 TCID50 was nasally administered to five 5-week-old male hamsters. Also, as a positive control, the SARS-CoV-2 temperature-sensitive strain, A50-18 strain, was nasally administered at the same titer and the same dose. After 3 weeks, the serum obtained by partially collecting blood was evaluated for neutralizing activity against SARS-CoV-2 B-1 strain. The neutralizing activity was evaluated by a method of determining the presence or absence of infectious viruses by mixing serially diluted serum and SARS-CoV-2 of 100 TCID50, reacting the mixture for 1 hour, then adding the mixture to Vero cells, and observing CPE after culturing for 4 days. The maximum dilution ratio of the serum in which CPE was not observed and the infectivity of the virus could be neutralized was defined as a neutralizing antibody titer. The results are shown in Fig. 32.

Seroconversions of neutralizing activities were observed in all individual infected hamsters of A50-18 strain (Reference Example) as a positive control group. In the candidate strain 1 and the candidate strain 3, seroconversion of neutralizing activity was not observed under the administration conditions in the present test example, and thus it is suggested that administration at a higher dose is necessary in order to exhibit immunogenicity. On the other hand, in all of the candidate strain 4, 6 and 7, seroconversions of neutralizing activities were observed in some individuals or all individuals, and it was found that these candidate strains had immunogenicity even at a low dose as in the present test example. In particular, in the hamster infected with the candidate strain 7 (Example), seroconversions of neutralizing activities were observed in all the individuals as with A50-18 strain, and thus the degree of immunogenicity was particularly excellent. That is, among the candidate strains in which seroconversions of neutralizing activities were observed under the administration conditions in the present test example, the candidate strain 4 and 6 were constructed by combining temperature-sensitive mutations and other attenuating mutations, so as to improve the safety, and in particular, the candidate strain 7 was constructed by combining temperature-sensitive mutations and growth reducing or other attenuating mutations, so as to maintain excellent immunogenicity even though the growth in the body was remarkably reduced.

### [Test Example 12] Evaluation of immunogenicity at low titer and low dose of candidate strain 7 (challenge test) (Example)

In order to evaluate whether the immunity induced in the candidate strain 7 contributes to the protection against infection, hamsters (male, 8 weeks old) after immunization were challenged by nasally administering 100 µL of 3 × 10⁵ TCID50 SARS-CoV-2 B-1 strain. The weight changes of the hamsters at that time are shown in Fig. 33. Similar to A50-18 strain (Reference Example) used as a positive control, the hamster infected with the candidate strain 7 (Example) did not lose weight when infected with SARS-CoV-2 B-1 strain. From this result, it was revealed that the candidate strain 7 (Example) could induce immunity that contributes to protection against infection similarly to A50-18 strain (Reference Example).

### [Test Example 13] Evaluation of immunogenicity at high titer and high dose of candidate strains 2 and 5 (Examples)

The immunogenicity of the candidate strains 2 and 5 (Examples) when tested at high titer and high dose was evaluated. To five 5-week-old male hamsters, 20 µL of the candidate strains of 1 × 10³ or 1 × 10⁴ TCID50 were nasally administered. In addition, as a positive subject, the SARS-CoV-2 temperature-sensitive strain, A50-18 strain (Reference Example) of 1 × 10³ TCID50 was nasally administered at the same dose. Blood was partially collected from hamsters recovered from infection after 3 weeks, and neutralizing activity of the obtained serum was evaluated. The neutralizing activity was measured by the same method as in Test Example 3. The results are shown in Fig. 34.

In the candidate strain 2, seroconversion of neutralizing antibodies were observed in four out of five individuals by administration of 1 × 10³ TCID50, and seroconversion of neutralizing antibodies were observed in all individuals when 1 × 10⁴ TCID50 was administered. In the candidate strain 5, the neutralizing activity of the serum was confirmed only in one out of five individuals by the administration of 1 × 10³ TCID50, but induction of neutralizing antibodies was observed in three out of five individuals by administering the virus of 1 × 10⁴ TCID50. From these results, it was revealed that the vaccine candidate strains in which the temperature-sensitive mutation(s) and the growth reducing or other attenuating mutation(s) were combined had low immunogenicity at the same dose as compared with the strains having only the temperature-sensitive mutation(s), but exhibited immunogenicity necessary for induction of neutralizing antibodies by increasing the dose. In addition, it was found that the candidate strain 2 showed immune induction since neutralizing activity was confirmed also in monkeys.

### [Test Example 14] Evaluation of growth of candidate strain 2 at each temperature

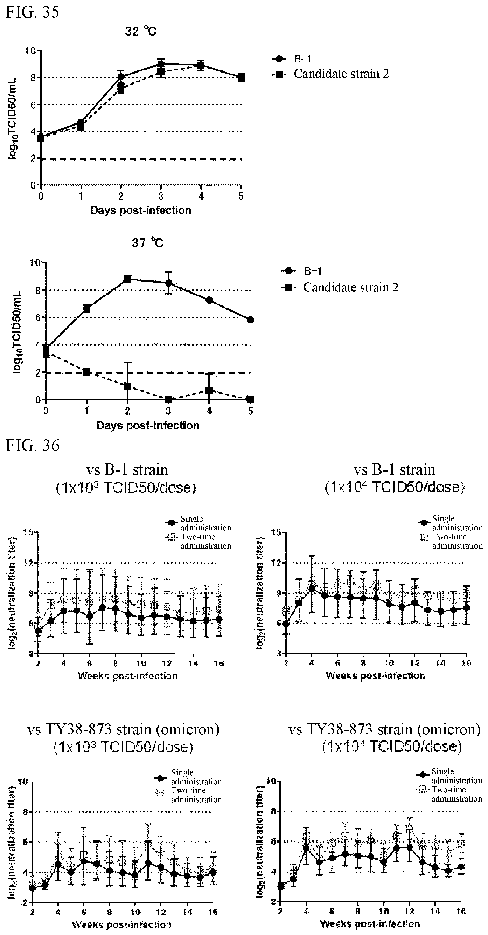

Vero cells were infected with the clinical isolate (B-1 strain [Comparative Example]) and the candidate strain 2 ([Example]) using 6-well plates under conditions of MOI = 0.01 (N = 3). The cells were cultured at 37°C or 32°C, and each culture supernatant was collected on 0 to 5 dpi. Virus titers of culture supernatants on 0 to 5 dpi were measured by TCID50/mL using the Vero cells. The results are shown in Fig. 35.

From Fig. 35, the candidate strain 2 ([Example]) grew at 32°C to the same extent as the clinical isolate (B-1 strain [Comparative Example]), whereas the growth at 37°C was remarkably reduced.

### [Test Example 15] Durability test of humoral immunity induced by administration of the candidate strain 2 (Example)

After 4-week-old male Syrian hamsters (n = 10) were bred for 1 week, the candidate strain 2 (Example) (1 × 10³ TCID50 or 1 × 10⁴ TCID50) was nasally administered at a dose of 20 µL under anesthetic conditions. In the group of twice administration, at the time point of 4 weeks after the first administration, the candidate strain 2 (1 × 10³ TCID50 or 1 × 10⁴ TCID50) was nasally administered again at a dose of 20 µL under anesthetic conditions. Blood was partially collected over time, and the obtained serum was heat-treated at 56°C for 30 minutes to be inactivated. The inactivated serum was serially diluted and mixed with B-1 strain (D614G type: pre-alpha European strain) or TY38-873 strain (Omicron variant) of 100 TCID50, and the mixture was reacted at 37°C for 1 hour. After the reaction, the culture mixture was seeded on Vero cells, and after culturing at 37°C, neutralizing activity of the virus was evaluated by observing CPE. The lowest dilution rate which did not cause CPE was defined as a neutralizing antibody titer. The results are shown in Fig. 36.

From Fig. 36, the neutralizing antibody titer of the serum increased to 6 to 12 by single administration of the candidate strain 2. In addition, when the second administration was carried out at the time point of 4 weeks after the first administration, an increase in neutralizing antibody titer was not observed, so as to suggest that sufficient humoral immunity was induced by single administration. Moreover, the serum neutralizing antibodies thus obtained were not significantly reduced even at 4 months after administration. From these results, it was revealed that the candidate strain 2 induced sufficient humoral immunity with a single administration of 1 × 10³ TCID50 in the hamster model, and the humoral immunity was lasting for at least 4 months after administration. In addition, by stimulating spleen cells of the candidate strain 2-infected hamsters with a spike antigen peptide, production of antigen-specific IFN-γ was induced. From this result, it was revealed that antigen-specific Th1 cells were induced by administration of the candidate strain 2, and that the candidate strain 2 also induced cellular immunity.

### [Test Example 16] Infection protection test by administration of the candidate strain 2

### (Example)

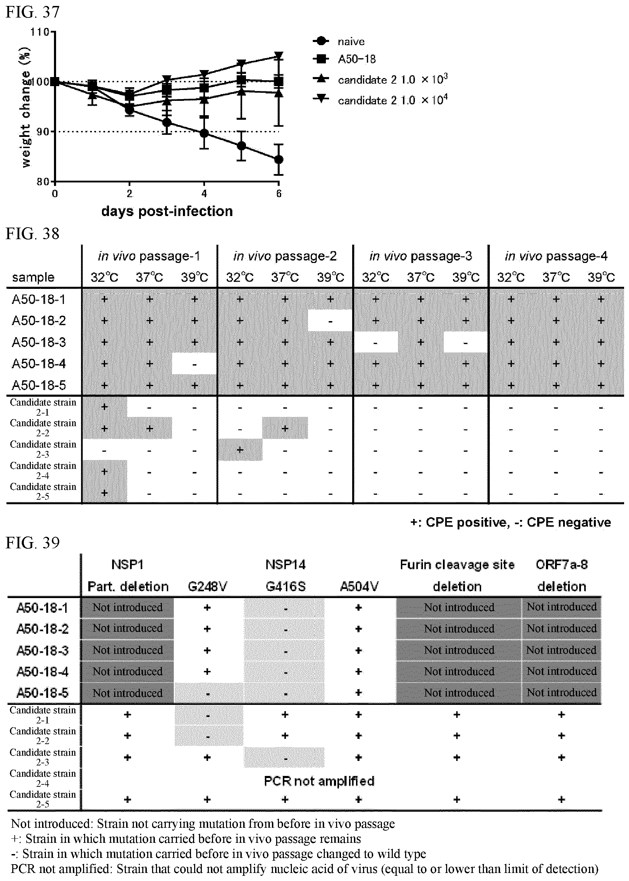

The hamsters to which 20 µL of the candidate strain 2 of 1 × 10³ or 1 × 10⁴ TCID50 or the SARS-CoV-2 temperature-sensitive strain, A50-18 strain, of 1 × 10³ TCID50 was nasally administered in Test Example 13 were challenged by nasally administering SARS-CoV-2 B-1 strain (3 × 10⁵ TCID50) at a dose of 100 µL under anesthetic conditions 3 weeks after the first administration. The weight of the hamsters after challenge were measured up to 6 days after administration. The results are shown in Fig. 37.

From Fig. 37, it was revealed that the hamsters infected with the candidate strain 2 or A50-18 strain did not lose weight at the time of infection with SARS-CoV-2 B-1 strain, and could induce immunity that contributes to protection against infection.

### [Test Example 17] Study on reversion of virulence during in vivo passage of the candidate strain 2 (Example)

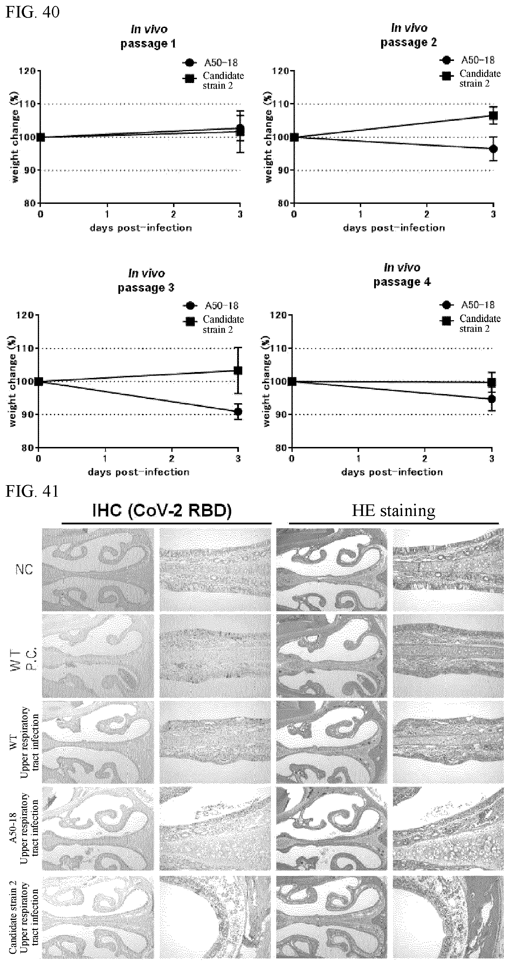

After 4-week-old male Syrian hamsters (n = 5) were bred for 1 week, the temperature-sensitive strain, A50-18 strain, or the candidate strain 2 (1 × 10⁵ TCID50) was nasally administered at a dose of 100 µL under anesthetic conditions. This dose is a human equivalent dose with a divisor of 30 extrapolating the human no adverse effect level from the hamster no adverse effect level, and corresponds to 2 × 10⁵ PFU/dose. The weight changes were observed, and the hamsters were euthanized 3 days after administration, and then nasal wash was collected using 500 µL of PBS. The obtained nasal wash was filtered and sterilized with a 0.22 µm filter, and then 100 µL of the nasal wash was nasally administered to next generation hamsters under anesthetic conditions. By performing the same operation three times, nasal wash when one to four in vivo passages were carried out was obtained. The obtained nasal wash was seeded on Vero cells and cultured at each temperature to evaluate the presence or absence of infectious viruses and the temperature sensitivity of the viruses. In addition, viral RNA was extracted from the nasal wash, and the base sequence of the target site was confirmed by Sanger sequencing method. The presence or absence of CPE in the Vero cells seeded with the nasal wash after each passage is shown in Fig. 38, the sequence confirmation results of the viral RNA extracted from the nasal wash when the fourth in vivo passage was carried out are shown in Fig. 39, and the results of weight changes of the hamsters at each in vivo passage are shown in Fig. 40.

Since A50-18 strain is a temperature-sensitive strain, CPE was not caused when cultured at 37°C or 39°C after infected to the Vero cells, but from Fig. 38, it was observed that CPE was caused when the nasal wash of the hamsters infected with A50-18 strain was added to the Vero cells and the cells were cultured at 37°C or 39°C. In addition, from Fig. 39, as a result of confirming the sequence of the viral RNA contained in the nasal wash after the in vivo passage, it was revealed that among the mutations in NSP14, which are mutations causing temperature sensitivity, G416S and G248V mutations were lost. Furthermore, from Fig. 40, it was observed that, when the weight changes of the hamsters at in vivo passage were evaluated, the weights of the hamsters infected with the nasal wash after in vivo passage of A50-18 strain decreased. From these results, it was suggested that the A50-18 strain had a possibility that the mutations causing temperature sensitivity were lost in the hamster body, so that a virulence-reversed strain that lost temperature sensitivity was generated and transmitted to another individual.

On the other hand, as to the candidate strain 2, from Fig. 38, viruses showing temperature sensitivity were detected from the nasal wash after the first in vivo passage, whereas viruses were hardly detected from the nasal wash after the second and subsequent in vivo passages. In addition, from Fig. 39, as a result of confirming the sequence of viral RNA contained in the nasal wash after the in vivo passage, there was a case where a part of the mutations causing temperature sensitivity was lost (however, even in such a passaged strain, only one of the combinations of mutations at positions 248 and 416 in NSP14 was lost). On the other hand, partial deletion of NSP1 (growth reducing mutation), deletion of furin cleavage site in Spike, and deletion of ORF7a-8, which were mutations that cause attenuation, were maintained. Furthermore, from Fig. 40, the decrease in weight was not observed even in the hamsters infected with the nasal wash after the in vivo passage of the candidate strain 2. From these results, it was suggested that the candidate strain 2 would be a candidate strain that was less likely to cause reversion of virulence compared with A50-18 strain that only had the mutations causing temperature sensitivity. From this result, it can be presumed that the betacoronavirus attenuated strain of the present invention has remarkably improved usefulness as a vaccine from the viewpoint that there is a low possibility that the virulence-reversed virus will transmit even when the virus is inoculated into a human as a live vaccine because prescribed temperature-sensitive mutations (substitution mutations) are further combined with prescribed growth reducing or other attenuating mutations (deletion mutations).

### [Test Example 18] Evaluation of tissue damage by the candidate strain 2 (Example)

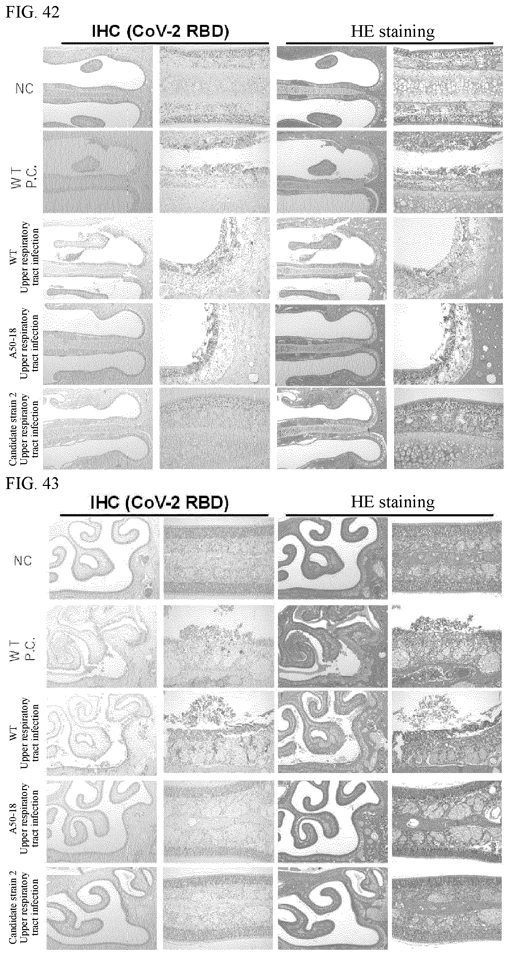

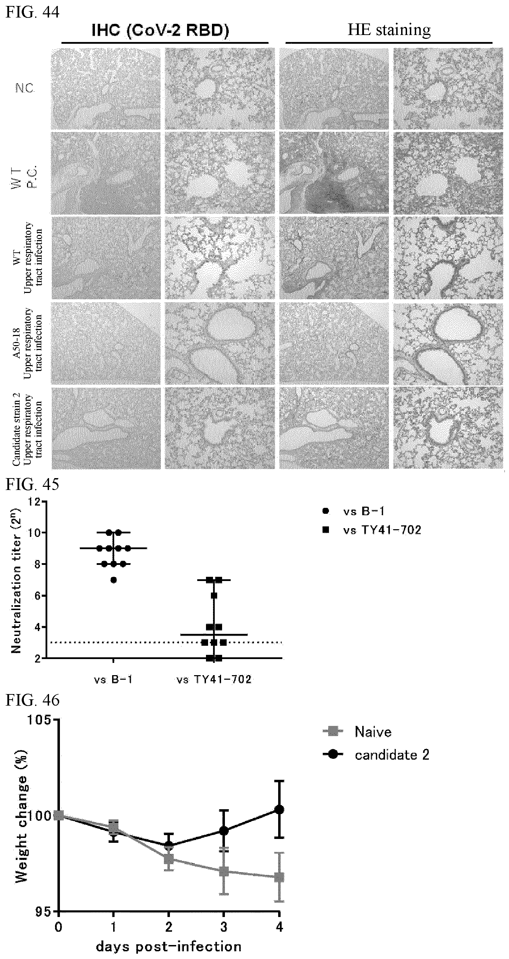

After 4-week-old male Syrian hamsters (n = 4) were bred for 1 week, SARS-CoV-2 B-1 strain [Comparative Example], the temperature-sensitive strain, A50-18 strain [Reference Example], or the candidate strain 2 [Example] (1 × 10⁵ TCID50) was nasally administered with a dose of 20 µL under anesthetic conditions. This dose is a human equivalent dose with a divisor of 30 extrapolating the human no adverse effect level from the hamster no adverse effect level, and corresponds to 4 × 10⁴ PFU/dose. The non-infected group (naive) was nasally administered with the same dose of a culture medium under anesthetic conditions. Also, as a positive control, SARS-CoV-2 B-1 strain (1 × 10⁵ TCID50) was nasally administered at a dose of 100 µL under anesthetic conditions. The virus fluid reached the upper respiratory tract of the hamster by nasal administration at a dose of 20 µL, and the virus fluid reached the lower respiratory tract of the hamster by nasal administration at a dose of 100 µL. Three days after administration, the hamsters were euthanized, and then the head and lungs were fixed with formalin. Tissue damages were evaluated by HE staining, and viral antigens were detected by IHC staining using Rabbit anti-spike RBD antibodies (Sino Biological (40592-T62)). Lesions in the nasal cavity and lung part of each individual, and scores for detection of viral antigens by IHC are shown in Table 6. In the table, Level 1 shows the tip of the nasal cavity, Level 2 shows the middle of the nasal cavity, and Level 3 shows the back of the nasal cavity. In addition, for representative examples in each site of each virus-infected hamster, Level 1 is shown in Fig. 41, Level 2 is shown in Fig. 42, Level 3 is shown in Fig. 43, and the lungs are shown in Fig. 44.

From Figs. 41 to 44, in the system in which SARS-CoV-2 B-1 strain (Comparative Example) was administered, not only in the positive control in which the virus fluid was nasally administered to the lower respiratory tract but also in the hamsters in which the virus fluid was nasally administered to the upper respiratory tract, tissue damage and viral antigen were detected in the deep part from the tip of the nasal cavity and in the lung. In the hamster to which A50-18 strain (Reference Example) or the candidate strain 2 (Example) was nasally administered to the upper respiratory tract, slight tissue damage and viral antigen were detected only in the tip of the nasal cavity, whereas tissue damage and viral antigen were not detected in the deep part of the nasal cavity and the lung. From these results, it was strongly suggested that in the candidate strain 2 (Example) and the temperature-sensitive strain, A50-18 strain (Reference Example), virus growth and associated tissue damage occurred in the tip of the nasal cavity close to the outside temperature, whereas virus growth and associated tissue damage were suppressed in the deep part of the nasal cavity and the lung close to body temperature, and that the candidate strain 2 (Example) had a higher inhibitory effect on virus growth and associated tissue damage, and had a lower risk of olfactory dysfunction.

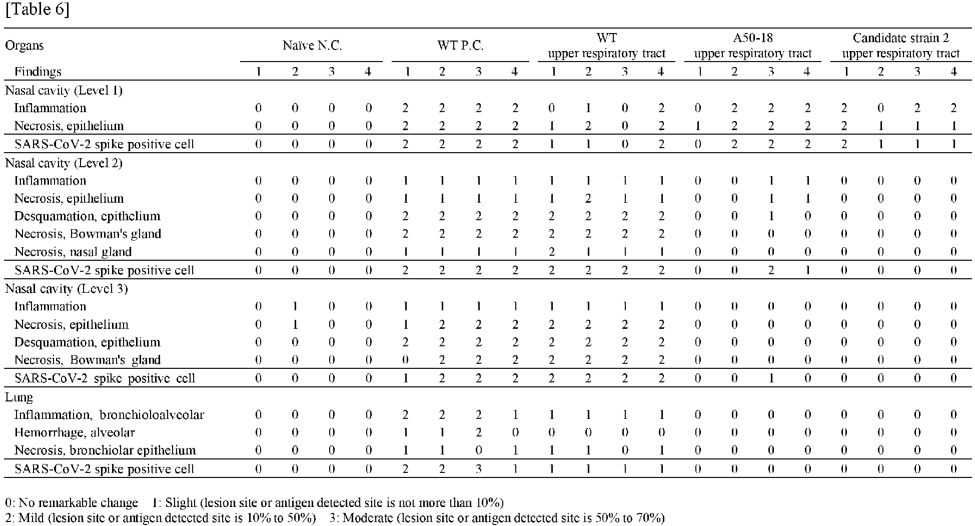

### [Test Example 19] Evaluation of immunogenicity by the candidate strain 2 (challenge with hetero strain)

It was evaluated whether the immunity induced by the candidate strain 2 contributed to the protection against infection of a SARS-CoV-2 Omicron variant. After 4-week-old male Syrian hamsters were bred for 1 week, the candidate strain 2 (1 × 10³ PFU) was nasally administered at a dose of 20 µL under anesthetic conditions. At the time point of 4 weeks after the administration, the serum was collected by partial blood collection. The obtained serum was heat-treated at 56°C for 30 minutes to be inactivated. The inactivated serum was serially diluted and mixed with B-1 strain (D614G type: pre-alpha European strain) or TY41-702 strain (Omicron variant: BA.5) of 100 TCID50, and the mixture was reacted at 37°C for 1 hour. After the reaction, the culture mixture was seeded on Vero cells, and after culturing at 37°C, neutralizing activity of the virus was evaluated by observing CPE. The lowest dilution rate which did not cause CPE was defined as a neutralizing antibody titer. The results are shown in Fig. 45. In addition, at the time point of 4 weeks after the administration, the candidate strain 2-administered hamsters and the naive hamsters were challenged by nasally administering TY41-702 strain (3 × 10⁵ PFU) at a dose of 100 µL under anesthetic conditions. The weights were measured over time, and at 4 days post-challenge, the hamsters were euthanized, and then the infectious viruses in the lungs and nasal wash were quantified. The weight changes at that time are shown in Fig. 46.

From Fig. 45, as a result of measurement of the neutralizing antibody titers, it was revealed that by administering the candidate strain 2, not only the neutralizing antibody against B-1 strain but also the neutralizing antibody against the Omicron variant BA.5 were induced to some extent. Furthermore, when the hamsters to which the candidate strain 2 was administered were challenged with the Omicron variant BA.5, the recovery of weight tended to be faster than that of the naive hamsters from Fig. 46. In addition, as a result of quantifying the viral amounts in the lungs and nasal wash 4 days after infection, it was suggested that the viral amount in the body of the hamsters to which the candidate strain 2 was administered was smaller than that of the naive hamsters.

### [Test Example 20] Evaluation of immunogenicity by the candidate strain 2 (evaluation of neutralizing activity by hetero strains)

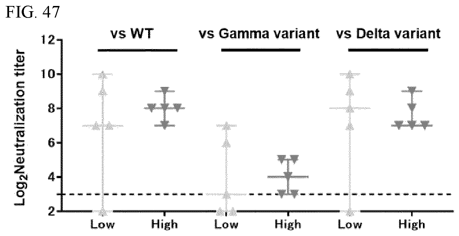

It was evaluated whether the immunity induced by the candidate strain 2 was effective against a SARS-CoV-2 delta variant and a gamma variant. Neutralizing activities against the gamma variant and the delta variant of the candidate strain 2 immune serum obtained in Test Example 13 were measured. The inactivated serum was serially diluted and mixed with BK325 strain (a delta variant) or TY7-501 strain (a gamma variant) of 100 TCID50, and the mixture was reacted at 37°C for 1 hour. After the reaction, the culture mixture was seeded on Vero cells, and after culturing at 37°C, neutralizing activity of the virus was evaluated by observing CPE. The lowest dilution rate which did not cause CPE was defined as a neutralizing antibody titer. The results are shown in Fig. 47 together with the evaluation of the neutralizing activity against the wild-type strain in Test Example 13. In Fig. 47, "Low" is a result of nasal administration of 20 µL of the candidate strain of 1 × 10³ TCID50, and "High" is a result of nasal administration of 20 µL of the candidate strain of 1 × 10⁴ TCID50.

From Fig. 47, it was shown that the immunity induced by the candidate strain 2 showed neutralizing activities against the gamma variant and the delta variant, and that the strain was effective. From these results, it was suggested that the immunity induced by the administration of the candidate strain 2 showed efficacy against the hetero strain variants.

## Claims

1. A betacoronavirus attenuated strain comprising:
non-structural protein(s) having the following mutation of (b), a combination of the following mutations of (e) and (f), and/or the following mutation of (h); and
structural protein(s), accessory protein(s), and/or non-structural protein(s) having the following mutations of (n), (o), and/or (r):
(b) a mutation of an amino acid residue corresponding to leucine at the 445th position of the amino acid sequence set forth in SEQ ID NO: 1 in NSP3,
(e) a mutation of an amino acid residue corresponding to glycine at the 248th position of the amino acid sequence set forth in SEQ ID NO: 2 in NSP14,
(f) a mutation of an amino acid residue corresponding to glycine at the 416th position of the amino acid sequence set forth in SEQ ID NO: 2 in NSP14,
(h) a mutation of an amino acid residue corresponding to valine at the 67th position of the amino acid sequence set forth in SEQ ID NO: 3 in NSP16,
(n) a loss-of-function mutation in ORF8,
(o) a deletion of an amino acid sequence corresponding to the 681st to 684th positions of the amino acid sequence set forth in SEQ ID NO: 4 in Spike, and
(r) a deletion of an amino acid sequence corresponding to the 32nd to 39th positions of the amino acid sequence set forth in SEQ ID NO: 8 in NSP1.

2. The virus attenuated strain according to claim 1, wherein four types of mutations or combination of mutations are selected from the following six types of mutations or combination of mutations: the mutation of (b), the combination of the mutations of (e) and (f), the mutation of (h), the mutation of (n), the mutation of (o), and the mutation of (r).

3. The virus attenuated strain according to claim 1, wherein one to two types of mutations or combination of mutations are selected from the following three types of mutations or combination of mutations: the mutation of (b), the combination of the mutations of (e) and (f), and the mutation of (h).

4. The virus attenuated strain according to claim 1, wherein the structural protein further contains the following mutations of (p) and/or (q):
(p) a mutation containing deletions of amino acid sequences corresponding to the 679th to 680th positions and the 685th to 686th positions of the amino acid sequence set forth in SEQ ID NO: 4 in Spike, and
(q) a mutation of an amino acid residue corresponding to valine at the 687th position of the amino acid sequence set forth in SEQ ID NO: 4 in Spike.

5. The virus attenuated strain according to claim 4, comprising the combination of the mutations of (e) and (f) and the following mutation of (g):
(g) a mutation of an amino acid residue corresponding to alanine at the 504th position of the amino acid sequence set forth in SEQ ID NO: 2 in NSP14.

6. The betacoronavirus attenuated strain according to claim 4, wherein the mutation of (n) is a deletion of an amino acid sequence corresponding to the amino acid sequence encoded by the base sequence set forth in SEQ ID NO: 7.

7. The virus attenuated strain according to claim 1, wherein the mutation of (b) is a substitution with phenylalanine, the mutation of (e) is a substitution with valine, the mutation of (f) is a substitution with serine, and the mutation of (h) is a substitution with isoleucine.

8. The virus attenuated strain according to claim 4, wherein the mutation of (q) is a substitution with isoleucine.

9. The virus attenuated strain according to claim 1, wherein the betacoronavirus is SARS-CoV-2 virus.

10. A live attenuated vaccine comprising the virus attenuated strain according to claim 1.

11. The live attenuated vaccine according to claim 10, which is administered nasally.

12. The live attenuated vaccine according to claim 10, which is administered intramuscularly, subcutaneously, or intradermally.
